(19) Europäisches Patentamt
European Patent Office
Office européen des brevets

(11) Veröffentlichungsnummer: **0 046 446**
**A1**

(12) # EUROPÄISCHE PATENTANMELDUNG

(21) Anmeldenummer: **81810320.2**

(22) Anmeldetag: **10.08.81**

(51) Int. Cl.³: **C 07 D 487/04**, C 07 D 471/14, A 61 K 31/415, A 61 K 31/505 // (C07D487/04, 239/00, 235/00),(C07D487/04, 239/00, 239/00),(C07D471/14, 239/00, 235/00, 221/00)

(30) Priorität: **15.08.80 CH 6197/80**

(43) Veröffentlichungstag der Anmeldung: **24.02.82 Patentblatt 82/8**

(84) Benannte Vertragsstaaten: **AT BE CH DE FR IT LI LU NL SE**

(71) Anmelder: **CIBA-GEIGY AG, Patentabteilung Postfach, CH-4002 Basel (CH)**

(72) Erfinder: **Frei, Jörg, Dr., Buechring 36, CH-4434 Hölstein (CH)**
Erfinder: **Schweizer, Ernst, Dr., Hollenweg 59, CH-4144 Arlesheim (CH)**

(54) **Neue polyazaheterocyclische Verbindungen, Verfahren zu ihrer Herstellung und diese enthaltende pharmazeutische Präparate.**

(57) Die vorliegende Erfindung betrifft neue polyazaheterocyclische Verbindungen der allgemeinen Formel I

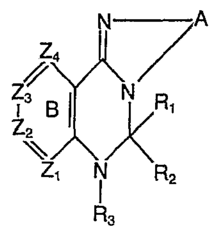

(I),

in welcher $R_1$ und $R_2$ unabhängig voneinander gegebenenfalls substituierte, niedere aliphatische Kohlenwasserstoffreste, gegebenenfalls substituiertes, direkt oder über Niederalkylen oder Niederalkenylen gebundenes Aryl oder Heteroaryl mit höchstens zwei Ringen, $R_3$ Wasserstoff oder Niederalkyl und A gegebenenfalls verzweigtes Niederalkylen oder Niederalkenylen mit 2 bis 4 Kohlenstoffatomen in direkter Kette zwischen den anliegenden Stickstoffatomen bedeuten und $Z_1$, $Z_2$, $Z_3$ und $Z_4$ Glieder des unsubstituierten oder substituierten Ringes B sind und gegebenenfalls entsprechend substituierte Reste –CH= bedeuten, eines jedoch auch den Rest –N= bedeuten kann, wobei $R_1$ und $R_2$ nicht beide Methyl oder beide Aethyl bedeuten, wenn $R_3$ Wasserstoff und A Aethenylen bedeutet und zugleich der Ring B unsubstituiert ist, und ihre Säureadditionssalze. Die Verbindungen der allgemeinen Formel I besitzen diuretische und antihypertensive Wirksamkeit, die bei solchen Verbindungen besonders ausgeprägt ist, in deren Rest A sich 3 oder 4 Kohlenstoffatome in direkter Kette zwischen den anliegenden Stickstoffatomen befinden. Verbindungen der allgemeinen Formel I, in deren Rest A sich 2 Kohlenstoffatome in direkter Kette zwischen den anliegenden Stickstoffatomen befinden, sind insbesondere antidiabetisch wirksam.

EP 0 046 446 A1

0046446

CIBA-GEIGY AG
Basel (Schweiz)

4-13013/+

Neue polyazaheterocyclische Verbindungen, Verfahren zu ihrer Herstellung und diese enthaltende pharmazeutische Präparate.

Die vorliegende Erfindung betrifft neue polyazaheterocyclische Verbindungen und ihre Säureadditionssalze, Verfahren zu ihrer Herstellung und diese enthaltende pharmazeutische Präparate, sowie ihre Verwendung.

Die erfindungsgemässen Verbindungen entsprechen der allgemeinen Formel I

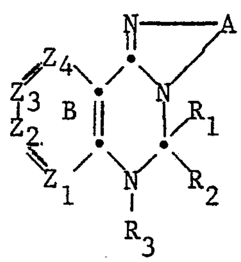

(I),

in welcher $R_1$ und $R_2$ unabhängig voneinander gegebenenfalls substituierte, niedere aliphatische Kohlenwasserstoffreste, gegebenenfalls substituiertes, direkt oder über Niederalkylen oder Niederalkenylen gebundenes Aryl oder Heteroaryl mit höchstens zwei Ringen, $R_3$ Wasserstoff oder Niederalkyl und A gegebenenfalls verzweigtes Niederalkylen oder Niederalkenylen mit 2 bis 4 Kohlenstoffatomen in direkter Kette zwischen den anliegenden Stickstoffatomen bedeuten und $Z_1$, $Z_2$, $Z_3$ und $Z_4$ Glieder des unsubstituierten oder substituierten Ringes B sind und gegebenenfalls entsprechend substituierte Reste -CH= bedeuten, eines jedoch auch den Rest -N= bedeuten kann, wobei $R_1$ und $R_2$ nicht beide Methyl oder beide Aethyl bedeuten, wenn $R_3$ Wasserstoff und A Aethenylen bedeutet und zugleich der Ring B unsubstituiert ist. Ebenfalls Gegenstand der Erfindung sind die Säureadditionssalze, insbesondere die pharmazeutisch annehmbaren Säureadditionssalze der Verbindungen der allgemeinen Formel I.

In den Verbindungen der allgemeinen Formel I enthalten vor- und nachstehend, soweit nichts anderes bemerkt, als nieder bezeichnete Reste höchstens 7 und vorzugsweise höchstens 4 Kohlenstoffatome.

Als niedere aliphatische Kohlenwasserstoffreste sind $R_1$ und/oder $R_2$ Niederalkyl, Niederalkenyl oder Niederalkinyl. Niederalkyl $R_1$ und/oder $R_2$ ist beispielsweise Pentyl, Isopentyl, Neopentyl, Hexyl, Isohexyl oder Heptyl, und vorzugsweise Methyl, Aethyl, Propyl, Isopropyl, Butyl oder Isobutyl. $R_1$ und $R_2$ mit dieser Bedeutung enthalten zusammen vorzugsweise mindestens 3 Kohlenstoffatome. Substituenten von Niederalkyl $R_1$ und/oder $R_2$ sind beispielsweise Niederalkoxy oder Niederalkylthio, wie Methoxy,Aethoxy, Propoxy, Isopropoxy, Butoxy oder Isobutoxy, bzw. Methylthio, Aethylthio, Propylthio, Isopropylthio oder Butylthio in beliebiger, vorzugsweise jedoch in 2-Stellung oder höherer Stellung des Niederalkyls, oder Niederalkoxycarbonyl, wie Methoxycarbonyl, Aethoxycarbonyl, Propoxycarbonyl, Isopropoxycarbonyl oder Butoxycarbonyl, in beliebiger Stellung, jedoch vorzugsweise in 1-Stellung des Niederalkyls. Als Niederalkenyl ist $R_1$ und/oder $R_2$ z.B. Vinyl, 1-Propenyl, Allyl, 1-Butenyl, 2-Methyl-1-propenyl, 2-Butenyl, 2-Methylallyl, 1-Pentenyl, 3-Methyl-1-butenyl, 3-Methyl-2-butenyl, 3,3-Dimethyl-1-butenyl, 1-Hexenyl oder 1-Heptenyl und als Niederalkinyl z.B. 2-Propinyl oder 2-Butinyl, die ebenfalls in der oben für Niederalkyl angegebenen Weise substituiert sein können.

Als Aryl- oder Heteroarylrest mit höchstens zwei Ringen ist $R_1$ beispielsweise Phenyl, 1- oder 2-Naphthyl, 5-gliedriges Heteroaryl, z.B. Furyl, wie 2- oder 3-Furyl, Thienyl, wie 2- oder 3-Thienyl, Pyrrolyl, wie Pyrrol-2-yl- oder 3-yl, Pyrazolyl, wie Pyrazol-3-yl,

-4-yl oder 5-yl, Isoxazolyl, wie 3-, 4- oder 5-Isoxazolyl, Isothiazolyl, wie 3-, 4- oder 5-Isothiazolyl, Oxazolyl, wie 2-, 4- oder 5-Oxazolyl, Thiazolyl, wie 2-, 4- oder 5-Thiazolyl, 6-gliedriges Diazaheteroaryl, z.B. Pyridazinyl, Pyrimidinyl, wie 2-, 4- oder 5-Pyrimidinyl, 2-Pyrazinyl, Benzoheteroaryl, z.B. Indolyl, wie 2-, 3- oder ar-Indolyl, Chinolinyl, wie 2-, 3-, 4- oder ar-Chinolinyl, Phthalazinyl, wie 1- oder ar-Phthalazinyl, Chinoxalinyl, wie 2- oder ar-Chinoxalinyl; Chianzolinyl, wie 2-, 4- oder ar-Chinazolinyl, oder insbesondere Pyridyl, wie 2-, 3- und vor allem 4-Pyridyl. Alle diese Reste können entweder über Niederalkylen, z.B. Propylen, Trimethylen, Tetramethylen, 1-Methyl- oder 2-Methyltrimethylen oder insbesondere Aethylen oder Methylen, ferner über Niederalkenylen, wie Propenylen, 1-Methyläthenylen und vor allem Aethenylen (Vinylen), und insbesondere direkt gebunden sein.

Als Substituenten von Aryl oder Heteroaryl $R_1$ und/oder $R_2$ kommen beispielsweise Niederalkyl, wie Aethyl, Propyl, Isopropyl, Butyl, Isobutyl, Tert.butyl und vor allem Methyl; Niederalkoxy, wie Aethoxy, Propoxy, Isopropoxy, Butoxy, Isobutoxy und vor allem Methoxy; Niederalkylthio, wie Aethylthio, Propylthio, Isopropylthio, Butylthio und vor allem Methylthio; weiter Halogen, insbesondere solches mit einer Atomnummer von höchstens 35, d.h. Fluor, Brom und insbesondere Chlor, Trifluormethyl, Methylendioxy, Hydroxy, Sulfamoyl, Nitro oder gegebenenfalls durch Niederalkyl mono- oder disubstituiertes oder durch Niederalkylen oder 3-Oxa-1,5-niederalkylen substituiertes Amino, wie Methyl-, Aethyl-, Propyl-, Isopropyl-, Butyl- oder Isobutylamino, Dimethyl- oder Diäthylamino bzw. 1-Pyrrolidinyl, Piperidino oder Morpholino, in Betracht. Solche Substituenten können ein- oder mehrfach, vorzugsweise höchstens dreifach, vorhanden und im letzteren Fall unter sich gleich oder verschieden sein, wobei jedoch Hydroxy und gegebenenfalls substituiertes Amino höchstens zweimal und vorzugsweise, wie auch Methylendioxy, höchstens einmal, Niederalkylthio und Trifluormethyl höchstens zweimal und die übrigen Substituenten vorzugsweise höchstens dreimal vertreten sein können.

$R_3$ ist als Niederalkyl beispielsweise Propyl, Butyl, Isobutyl, Pentyl, Isopentyl, Neopentyl, Hexyl, Heptyl und vor allem Methyl oder Aethyl.

Als gegebenenfalls verzweigtes Niederalkylen oder Niederalkenylen mit zwei Kohlenstoffatomen in direkter Kette zwischen den beiden Stickstoffatomen ist A z.B. Propylen (1-Methyläthylen), 1,1-Dimethyläthylen, 1,2-Dimethyläthylen, 1-Aethyläthylen und vor allem Aethylen, bzw. 1-Methyläthenylen, 1,2-Dimethyläthenylen und vor allem Aethenylen (Vinylen). Definitionsgemässe Reste A mit drei Kohlenstoffatomen zwischen den beiden Stickstoffatomen sind z.B. 1-Methyl- oder 2-Methyltrimethylen, 1,2-Dimethyl- oder 1,3-Dimethyltrimethylen, 1,1- oder 2,2-Dimethyltrimethylen, 2- oder 3-Methyl-propenylen, 2,3- oder 3,3-Dimethylpropenylen und vor allem Trimethylen oder Propenylen, und definitionsgemässe Reste A mit 4 Kohlenstoffatomen zwischen den beiden Stickstoffatomen sind z.B. 1- oder 2-Methyltetramethylen, 1,1- oder 2,2-Dimethyltetramethylen, 1,4- oder 2,3-Dimethyltetramethylen, 1,1- oder 2,3-Dimethyl-2-butenylen, und vor allem Tetramethylen oder 2-Butenylen.

Als Substituenten des Ringes B bzw. von dessen Ringgliedern $Z_1$ bis $Z_4$ als -CH= können dieselben wie im Aryl oder Heteroaryl $R_1$, im wesentlichen auch in derselben Anzahl vorliegen, wobei neben dem unsubstituierten Ring B insbesondere durch Niederalkyl, vor allem Methyl, durch Niederalkoxy, vor allem Methoxy, und/oder Halogen bis Atomnummer 35, vor allem Chlor, bis dreifach substituierte, aber vor allem monosubstituierte Ringe B und ferner auch durch andere der für Aryl oder Heteroaryl $R_1$ genannten Substituenten monosubstituierte Ringe B besonders in Betracht kommen.

Als Säureadditionssalze, insbesondere pharmazeutisch annehmbare Säureadditionssalze der Verbindungen der allgemeinen Formel I kommen beispielsweise solche mit geeigneten anorganischen Säuren, wie Halogenwasserstoffsäuren, z.B. Chlorwasserstoffsäure oder Bromwasserstoffsäure, ferner Salpetersäure, Schwefelsäure oder Phosphorsäure, oder mit geeigneten organischen Säuren, wie organischen Carbonsäuren, u.a. gegebenenfalls Hydroxy enthaltenden Niederalkan- oder Niederalkencarbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure oder Zitronensäure, oder Aryl-, Arylniederalkan-, Arylniederalken- oder Heterocyclylcarbonsäuren, z.B. Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, oder organischen Sulfonsäuren, wie gegebenenfalls Hydroxy enthaltenden Niederalkansulfonsäuren. z.B. Methansulfonsäure, Aethansulfonsäure, 2-Hydroxyäthansulfonsäure oder Aethan-1,2-disulfonsäure, oder Arylsulfonsäuren, z.B. Benzolsulfonsäure, 4-Methyl-benzolsulfonsäure oder Naphthalin-2-sulfonsäure, oder weiteren sauren Substanzen, wie Ascorbinsäure, in Frage. Entsprechend der engen Beziehung zwischen den freien Basen und ihren Säureadditionssalzen beziehen sich nachstehend, soweit sinngemäss, Angaben betreffen freie Basen auch auf Säureadditionssalze und umgekehrt Angaben betreffend Säureadditionssalze auch auf freie Basen.

Die neuen polyazaheterocyclischen Verbindungen der allgemeinen Formel I und ihre Additionssalze mit anorganischen und organischen Säuren besitzen wertvolle pharmakologische Eigenschaften. Sie wirken insbesondere diuretisch und natriuretisch an der Ratte im Dosisbereich von 10 bis 1000 mg/kg per os und am Hund im Dosisbereich von 5 bis 50 mg/kg per os, wie durch Sammeln des Harns während 3 Stunden nach Verabreichung (Ratte) bzw. stündlich während 5 Stunden nach Verabreichung (Hund) und Bestimmung des Harnvolumens und der Natrium-, Kalium- und Chlorionen festgestellt werden kann. Hierbei zeichnen sich die Verbindungen der Formel I durch fehlende Beeinflussung der Kalium-Ausscheidung aus, hervorzuheben ist auch die gute Verträglichkeit. Besonders ausgeprägt ist die diuretische Wirksamkeit bei denjenigen Verbindungen der allgemeinen Formel I, in denen A gegebenenfalls verzweigtes Niederalkylen mit 4 oder insbesondere 3 Kohlenstoffatomen in direkter Kette zwischen den anliegenden Stickstoffatomen, wor allem Trimethylen bedeutet. Entsprechend können die Verbindungen der allgemeinen Formel I, insbesondere diejenigen der vorgenannten Art, und ihre pharmazeutisch annehmbaren Säureadditionssalze als kaliumneutrale Diuretica und Antihypertensiva verwendet werden.

Diejenigen Verbindungen der allgemeinen Formel I, in denen der Rest A zwei Kohlenstoffatome in direkter Kette zwischen den beiden Stickstoffatomen aufweist, besitzen auch hypoglykämische Wirksamkeit, wie sich an Stoffwechsel-normalen Ratten nach oraler Verabreichung von Dosen ab 3 mg/kg sowie auch an Ratten, die durch Injektion von Streptozotocin in eine Diabetes-ähnliche Stoffwechsellage versetzt wurden [vgl.A.Junod et al., Proc.Soc.Exp.Biol.Med. 126, 201-205 (1967)], nachweisen lässt. Demgegenüber ist ihre diuretische Wirksamkeit weniger ausgeprägt. Die Senkung des Blutzuckerspiegels ist weder von einer Hyperlactatämie noch, bei normal gefütterten Ratten, von einer Erhöhung des Plasmainsulins begleitet. Die pharmakologischen Befunde charakterisieren solche Verbindungen der allgemeinen Formel I und ihre pharmazeutisch annehmbaren Säureadditionssalze als Antidiabetica, die zur oralen Behandlung von Hyperglykämie bei Säugetieren, insbesondere von Diabetes mellitus, verwendet werden können.

Die Verbindungen der allgemeinen Formel I besitzen überdies antiphlogistische und antinociceptive Wirksamkeit, die bei oraler Verabreichung im Kaolinödem-Test an der Rattenpfote bzw. im Writhing Syndrom-Test an der Maus nachgewiesen werden können. Sie wirken auch lipidsenkend, wie z.B. bei der männlichen Ratte bei Verabreichung von je einmal 50 mg/kg an drei aufeinanderfolgenden Tagen und zweimal 50 mg/kg am vierten Tag in dem am fünften Tag gewonnenen Serum an der Senkung der Lipoproteine von sehr geringer Dichte (very-low-density lipoproteins = VLDL) und der Triglyceride feststellbar ist.

Die Erfindung betrifft insbesondere polyazaheterocyclische Verbindungen der allgemeinen Formel I, in denen $R_1$ und $R_2$ Reste entsprechend der unter der Formel I angegebenen Definition bedeuten, die

unsubstituiert oder in der oben näher angegebenen Weise substituiert sind und zusammen und einschliesslich der gegebenenfalls vorhandenen Substituenten vorzugsweise 3 bis 20 Kohlenstoffatome und insbesondere 5 bis 15 Kohlenstoffatome aufweisen, wobei $R_1$ und $R_2$ als Aryl vor allem Phenyl und als Heteroaryl vor allem Pyridyl, Thienyl oder Furyl bedeuten, welche Reste unsubstituiert oder, wie oben angegeben, substituiert sind, $R_3$ Wasserstoff oder Niederalkyl mit höchstens 4 Kohlenstoffatomen und A unsubstituiertes oder durch Niederalkyl, insbesondere ein oder zwei Methyl, substituiertes Aethylen oder Trimethylen mit insgesamt höchstens 4 bzw. 5 Kohlenstoffatomen, Aethenylen, Propenylen oder Tetramethylen bedeutet, die Reste $Z_1$, $Z_2$, $Z_3$ und $Z_4$ die unter der Formel I angegebene Bedeutung haben, und der Ring B unsubstituiert oder in der weiter oben näher angegebenen Weise substituiert ist, und deren Säureadditionssalze, insbesondere die pharmazeutisch annehmbaren Säureadditionssalze. Vor allem betrifft die Erfindung Verbindungen der allgemeinen Formel I, in denen $R_1$ monoheterocyclisches monocyclisches Heteroaryl, das unsubstituiert oder durch Niederalkyl, Niederalkoxy und/oder Halogen bis Atomnummer 35 substituiert ist, und über Methylen oder vorzugsweise direkt gebunden ist, insbesondere unsubstituiertes oder entsprechend, vor allem durch Niederalkyl, wie Methyl, substituiertes Pyridyl, oder entsprechend substituiertes oder vor allem unsubstituiertes Thienyl oder Furyl, $R_2$ einen ebensolchen Rest, insbesondere unsubstituiertes oder entsprechend substituiertes Pyridyl, oder entsprechend substituiertes oder vor allem unsubstituiertes Thienyl, oder unsubstituiertes oder, wie vorstehend für monocyclisches Heteroaryl $R_1$ angegeben, substituiertes Phenyl oder unsubstituiertes oder, wie weiter oben angegeben, substituiertes Niederalkyl, insbesondere unsubstituiertes Niederalkyl oder Niederalkoxycarbonylniederalkyl, vor allem Niederalkoxycarbonylmethyl, $R_3$ Wasserstoff oder Alkyl mit höchstens 4 Kohlenstoffatomen, insbesondere Methyl oder Aethyl, und A Aethylen oder Trimethylen bedeutet, die Reste $Z_1$, $Z_2$, $Z_3$ und $Z_4$ die unter der Formel I angegebene Bedeutung haben und der Ring B in der vorstehend für $R_1$ näher angegebenen Weise, insbesondere aber durch Halogen bis Atomnummer 35, wie

Chlor, in erster Linie am Ringglied $Z_2$ substituiert oder vor allem unsubstituiert ist, und deren Säureadditionssalze, insbesondere die pharmazeutisch annehmbaren Säureadditionssalze.

In erster Linie betrifft die Erfindung Verbindungen der allgemeinen Formel I, in denen $R_1$ unsubstituiertes oder durch Niederalkyl, insbesondere Methyl, substituiertes Pyridyl, oder Thienyl, insbesondere 2-Thienyl, $R_2$ einen ebensolchen Rest oder durch Niederalkyl, Niederalkoxy oder Halogen bis Atomnummer 35 substituiertes oder insbesondere unsubstituiertes Phenyl, Niederalkyl oder Niederalkoxycarbonylniederalkyl, insbesondere Niederalkoxycarbonylmethyl, $R_3$ Wasserstoff oder Niederalkyl, insbesondere Methyl oder Aethyl, A Aethylen oder Trimethylen, $Z_1$, $Z_2$, $Z_3$ und $Z_4$ Reste -CH= bedeuten, von denen $Z_2$ durch Halogen bis Atomnummer 35, insbesondere Chlor, substituiert sein kann und somit der Ring B unsubstituiert oder entsprechend substituiert ist, und deren pharmazeutisch annehmbare Säureadditionssalze.

Verbindungen vom zuletzt angegebenen Typus, in denen A Aethylen bedeutet, sind z.B. die nachstehenden, welche bei oraler Verabreichung an jeweils in Klammern in mg/kg angegebenen Dosis an der Ratte eine Senkung des Blutzuckerspiegels um mindestens 20% bewirken: 5-Methyl-5-(4-pyridyl)-2,3,5,6-tetrahydroimidazo[1,2-c]-chinazolin (10), 5-Methyl-5-(6-methyl-2-pyridyl)-2,3,5,6-tetrahydroimidazo[1,2-c]-chinazolin (3), 5-Butyl-5-(4-pyridyl)-2,3,5,6-tetrahydro-imidazo-[1,2-c]chinazolin (10), 5-Methyl-5-(2-pyridyl)-8-chlor-2,3,5,6-tetrahydro-imidazo[1,2-c]chinazolin (3), 5-Phenyl-5-(4-pyridyl)-2,3,5,6-tetrahydro-imidazo[1,2-c]chinazolin (10), 5-(4-Pyridyl)-2,3,5,6-tetrahydroimidazo[1,2-c]chinazolin-5-essigsäureäthylester (10) und 5-Propyl-5-(4-pyridyl)-2,3,5,6-tetrahydro-imidazo[1,2-c]chinazolin (3).

Verbindungen vom zuletzt angegebenen Typus, in denen A Trimethylen bedeutet, sind z.B. die nachstehend genannten, welche an weiblichen Hunden (Tierzahl = n) in der Dosierung von 20 mg/kg per os während der ersten 5 Stunden nach Verabreichung folgende

durchschnittliche stündliche Ausscheidung von Natrium-, Kalium- und Chlorionen und des Harnvolumens (= Vol.), in Prozenten der in der Stunde unmittelbar vor Verabreichung von den gleichen Tieren ausgeschiedenen Mengen ausgedrückt, ergeben: 6-Phenyl-6-(4-pyridyl)-3,4,6,7-tetrahydro-2H-pyrimido[1,2-c]chinazolin (n = 4, $Na^+$ 1224, $K^+$ 114, $Cl^-$ 1369, Vol. 409); 6-Phenyl-6-(2-pyridyl)-3,4,6,7-tetrahydro-2H-pyrimido[1,2-c]-chinazolin (n = 2, $Na^+$ 392, $K^+$ 56, $Cl^-$ 308, Vol. 171); 6-(4-Methylphenyl)-6-(4-pyridyl)-3,4,6,7-tetrahydro-2H-pyrimido[1,2-c]chinazolin-methansulphonat (n = 2, $Na^+$ 800, $K^+$ 103, $Cl^-$ 850, Vol. 206); 6-(4-Chlorphenyl)-6-(4-pyridyl)-3,4,6,7-tetrahydro-2H-pyrimido[1,2-c]chinazolin (n = 4, $Na^+$ 1673, $K^+$ 92, $Cl^-$ 868, Vol. 301); 6-(4-Pyridyl)-6-(2-thienyl)-3,4,6,7-tetrahydro-2H-pyrimido[1,2-c]chinazolin (n = 4, $Na^+$ 1738, $K^+$ 100, $Cl^-$ 1233, Vol. 514); 7-Aethyl-6-phenyl-6-(4-pyridyl)-3,4,6,7-tetrahydro-2H-pyrimido[1,2-c]chinazolin-fumarat-(1:1) (n = 4, $Na^+$ 721, $K^+$ 66, $Cl^-$ 889, Vol. 275). Bei der zuerst genanten Verbindung betragen die Ausscheidungen innerhalb der ersten 5 Stunden nach Verabreichung von 20 mg/kg per os in Prozenten der gleichzeitig für die gleiche Zeitdauer bestimmten Ausscheidungen von 10 unbehandelten Kontrolltieren für $Na^+$ 2263, $K^+$ 137, $Cl^-$ 751 und Vol. 379. Alle obigen Versuche zeigen einerseits die starke Steigerung der Ausscheidung von Natrium und Chlor, sowie die Steigerung des Harnvolumens, während andererseits die Kaliumausscheidung unverändert oder teilweise sogar vermindert ist.

Die Verbindungen der allgemeinen Formel I und ihre Säureadditionssalze können in an sich bekannter Weise hergestellt werden, beispielsweise indem man eine Verbindung der allgemeinen Formel II,

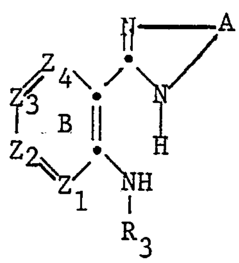

(II)

in welcher $R_3$, $Z_1$, $Z_2$, $Z_3$, $Z_4$ und A die unter der Formel I angegebene Bedeutung haben und der Ring B, wie dort angegeben, substituiert sein kann, oder ein Säureadditonssalz derselben mit einem Keton der allgemeinen Formel III.

$$R_1 - CO - R_2 \qquad \text{(III)}$$

in welcher $R_1$ und $R_2$ die unter der Formel I angegebene Bedeutung haben, oder einem reaktionsfähigen funktionellen Derivat desselben kondensiert, gewünschtenfalls in eine Verbindung der allgemeinen Formel I, in welcher $R_3$ Wasserstoff bedeutet, Niederalkyl als Rest $R_3$ einführt, und/oder eine erhaltene Verbindung der allgemeinen Formel I in ein Säureadditionssalz überführt oder aus einem erhaltenen Säureadditionssalz die Verbindung der allgemeinen Formel I freisetzt.

Die Kondensation einer Verbindung der allgemeinen Formel II mit einem Keton der allgemeinen Formel III oder einem reaktionsfähigen funktionellen Derivat eines solchen kann man z.B. in Gegenwart eines inerten organischen Lösungsmittels, z.B. in einem niederen Alkanol, wie Methanol, Aethanol, Isopropanol oder Butanol, in einem aromatischen Kohlenwasserstoff, wie Benzol, Toluol oder einem Xylol oder Xylolgemisch, oder in einem N-substituierten Säureamid, wie Dimethylformamid oder Hexamethylphosphorsäuretriamid, beispielsweise bei Temperaturen zwischen etwa 0° und etwa 180°C, vorzugsweise bei Raumtemperatur bis etwa 150°C und vorzugsweise in Gegenwart einer Säure oder eines sauren Katalysators durchführen. Als Säure kann beispielsweise eine Mineralsäure wie Chlorwasserstoff oder konz. Salzsäure, vorzugsweise in einem niederen Alkanol, weiter Polyphosphorsäure, z.B. in Dimethylformamid oder als alleiniges Reaktionsmedium, oder eine katalytische Menge einer Arensulfonsäure, wie p-Toluolsulfonsäure, vorzugsweise in einem aromatischen Kohlenwasserstoff, wie Toluol, insbesondere bei dessen Siedetemperatur unter Entfernung des freigesetzten Wassers durch azeotrope Destillation, verwendet werden. Als saure Katalysatoren kommen beispielsweise stark saure Ionenaustascherharze, wie z.B. Amberlit IR 120 $H^{\oplus}$-Form (geschützte Marke der Firma Rohm & Haas Co., Philadelphia, U.S.A.) in Betracht. Anstelle von sauren Katalysatoren kann man auch andere Mittel verwenden, welche die Freisetzung von Wasser fördern, wie Kieselgel oder Aluminiumoxid, und die Kondensation wiederum vorzugsweise in einem Lösungsmittel durchführen, das die Entfernung des freigesetzten Wasers durch azeotropes Destillation am Wasserabscheider ermöglicht, wie z.B. in Toluol bei dessen Siedetemperatur. Man kann ferner z.B. auch Polyphosphatester (PPE) verwenden.

Weiter kann die Umsetzung auch in Abwesenheit von Lösungs- oder Verdünnungsmitteln, aber vorzugsweise bei erhöhten Temperaturen zwischen ca. 120 und 200°C und ebenfalls in Gegenwart von Säuren oder sauren Kondensationsmitteln, wie z.B. einer Katalytischen Menge p-Toluolsulfonsäure, oder einer vorzugsweise mindestens äquimolaren Menge Aluminiumchlorid, dem ein festes Verdünnungsmittel, wie Natriumchlorid, beigefügt sein kann, durchgeführt werden. Wie aus vorstehendem und aus den Beispielen ersichtlich, können die Reaktionsbedingungen innerhalb weiter Grenzen variieren. Es ist klar, dass man nicht energischere Bedingungen anwendet, als zur Erzielung einer möglichst vollständigen Umsetzung notwendig sind. Im Rahmen des obengenannten Bereichs sind relativ energische Reaktionsbedingungen im allgemeinen nötig, wenn in den verwendeten Ausgangsstoffen der allgemeinen Formel III als $R_1$ und $R_2$ direkt gebundene cyclische Reste vorliegen.

Als reaktionsfähige funktionelle Derivate von Ketonen der allgemeinen Formel III können beispielsweise Alkoholate, wie Niederalkanolate, Ketale, wie Dimethyl-, Diäthyl- und Aethylenketale, oder Oxime sowie Imine verwendet werden.

Die Einführung von Niederalkyl anstelle eines Wasserstoffatoms $R_3$ kann in an sich bekannter Weise, beispielsweise durch Umsetzung einer Verbindung der allgemeinen Formel I, die als $R_3$ ein Wasserstoffatom aufweist, mit einem reaktionsfähigen Ester eines Niederalkanols, insbesondere einem entsprechenden Halogenwasserstoffsäure-, Niederalkansulfonsäure- oder Arensulfonsäure-ester, vor allem einem Niederalkylhalogenid, insbesondere -jodid oder -bromid, wie z.B. Methyl- oder Aethyljodid, Propyl- oder Butylbromid, in An- oder Abwesenheit eines organischen Lösungsmittels, z.B. eines Niederalkanols, wie Methanol, Aethanol oder Isopropanol, eines Niederalkanons, wie Aceton oder 2-Butanon, eines Niederalkansäureesters oder -amids, wie Aethylacetat bzw. Dimethylformamid, oder eines niedrigsiedenden Kohlenwasserstoffs oder Polyhalogenkohlenwasserstoffs wie Benzol, Toluol bzw. Methylenchlorid, und eines säurebindenden Mittels, z.B. einer Alkalimetallverbindung, wie Natriumhydrid, Natrium- oder Lithiumamid, einer tertiären organischen Base, wie Triäthylamin oder Aethyldiisopropylamin, oder einer anorganischen Base, wie Kalium- oder Natrium-carbonat, vorzugsweise bei Temperaturen zwischen 0°C und 120°C und nötigenfalls im geschlossenen Gefäss, insbesondere jedoch bei Raumtemperatur bis Siedetemperatur des Reaktionsgemisches erfolgen.

Von den Ausgangsstoffen der allgemeinen Formel II sind einzelne Vertreter bekannt und weitere analog zu den bekannten Verbindungen herstellbar. Beispielsweise können sie durch eine in der US-PS 3 920 687 für die Herstellung des 2-(2-Aminophenyl)-4,5-dihydro-1H-imidazol und des 2-(2-Aminophenyl)-4,5-dihydro-5,5-dimethyl-1H-imidazol und in der US-PS 3 922 282 für die Herstellung des 2-(2-Amino-4-chlorphenyl)-4,5-dihydro-1H-imidazol beschriebene Reaktionsfolge erhalten werden, die für die genannten Verbindungen in der N-Acylierung von Anthranilsäure- bzw. 4-Chloranthranilsäure-methylester mit p-Methoxybenzolsulfonylchlorid, Kochen der erhaltenen N-(p-Methoxybenzolsulfonyl)-derivate mit 1,2-Aethandiamin bzw. 2-Methyl-1,2-propandiamin, und Erwärmen der erhaltenen Derivate des 4,5-Dihydro-1H-imidazols mit 92%iger Schwefelsäure und Freisetzen der gewünschten Verfahrensprodukte mit überschüssiger wässriger Ammoniaklösung besteht. Das 2-(2-Aminophenyl)-4,5-dihydro-1H-imidazol wurde ferner gemäss Zh.Prikl.Khim. 43, 1641 (1970) (vgl. Chem. Abstr. 73, 77138r) durch Kondensation von Anthranilsäure mit 1,2-Aethandiamin in Gegenwart eines bestimmten Kationaustauscherharzes bei Raumtemperatur, und gemäss J.Med.Chem. 13, 697 (1970) durch katalytische Hydrierung

von 4,5-Dihydro-2-(2-nitrophenyl)-1H-imidazol oder 2-(2-Nitrophenyl)-1H-imidazol hergestellt, vgl. zu letzterem auch Il Farmaco, Ed.Sc. 30, 536-546, besonders 539 (1975). Als weiteres Verfahren kommt z.B. die Kondensation von unsubstituierten oder ringsubstituierten 2-Aminobenzonitrilen, oder von entsprechenden Aminopyridincarbonitrilen mit Amino- und Cyanogruppe an benachbarten Ringkohlenstoffatomen, mit gegebenenfalls C-niederalkyliertem 1,2-Aethandiamin in Betracht. Als Verbindungen der allgemeinen Formel II mit Niederalkyl als $R_3$ wurden das 4,5-Dihydro-2-[2-(methylamino)-phenyl]-1H-imidazol und das 4,5-Dihydro-2-[2-(äthylamino)-phenyl]-1H-imidazol gemäss Bull.Soc.Chim. France 1975, 2118-20 durch Umsetzung der entsprechenden 1-Niederalkyl-1,2-dihydro-3,1-benzothiazin-4-thione mit 1,2-Aethandiamin im siedenden Gemisch von Aethanol-Benzol (2:1) hergestellt.

Als Ausgangsstoffe der allgemeinen Formel II, in denen der Rest A drei bzw. vier Kohlenstoffatome in direkter Kette zwischen den beiden Stickstoffatomen enthält, wurden das 2-(2-Aminophenyl)-1,4,5,6-tetrahydropyrimidin und das 2-(2-Aminophenyl)-1,5,6,7-tetrahydro-1H-1,3-diazepin von Russell Kwok im J. Heterocyclic Chem. 15, 877-880 (1978) beschrieben. Weitere Verbindungen von diesem Typus können analog zum dort beschriebenen Verfahren, d.h. durch mehrstündiges Erhitzen von gegebenenfalls ringsubstituiertem 2-Aminobenzonitril mit je ungefähr den halbmolaren Mengen von gegebenenfalls C-niederalkyliertem 1,3-Propandiamin, bzw. 1,4-Butandiamin und deren Bis-4-methylbenzolsulfonaten auf etwa 200°C, hergestellt werden. Ferner kann man z.B. auch zu entsprechenden Ausgangsstoffen gelangen, indem man ein gegebenenfalls ringsubstituiertes 2-Aminobenzonitril, oder ein entsprechendes Aminopyridincarbonitril mit Amino- und Cyanogruppe an benachbarten Ringkohlenstoffatomen, mit der etwa doppelt molaren Menge von gegebenenfalls C-niederalkyliertem 1,3-Propandiamin und wenig Schwefelkohlenstoff einige Zeit, z.B. etwa 2 bis 48 Stunden, unter Rühren und Durchleiten von Stickstoff auf etwa 120 bis 170°C erhitzt.

Von den Ketonen der allgemeinen Formel III sind manche bekannt und weitere analog zu den bekannten herstellbar; Niederalkylpyridyl-ketone beispielsweise durch Umsetzung der entsprechenden Pyridincarbonitrile mit Niederalkylmagnesiumhalogeniden nach Grignard.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Salze der neuen Verbindungen können in an sich bekannter Weise, z.B. durch Behandeln mit basischen Mitteln, wie Alkalimetallhydroxiden, -carbonaten oder -hydrogencarbonaten, oder Ionenaustauschern, in die freien Verbindungen übergeführt werden. Andererseits können erhaltene freie Verbindungen in an sich bekannter Weise, z.B. durch Behandeln mit organischen oder anorganischen Säuren, wie den obgenannten Säuren, Säureadditionssalze bilden, wobei zur Herstellung insbesondere solche Säuren verwendet werden, die sich zur Bildung von pharmazeutisch verwendbaren Salzen eignen.

Diese oder andere Säureadditionssalze der neuen Verbindungen, wie z.B. Oxalate, Pikrate oder Perchlorate, können auch zur Reinigung der erhaltenen freien Basen dienen, indem man die freien Verbindungen in Salze überführt, diese abtrennt und reinigt, und aus den Salzen wiederum die Basen freisetzt.

Die neuen Verbindungen können, je nach der Wahl der Ausgangsstoffe und Arbeitsweisen, als Racemate oder optische Antipoden oder, sofern sie mindestens zwei Asymmetriezentren enthalten, auch als Gemische von Racematen vorliegen. Die Ausgangsstoffe können auch in Form von bestimmten optischen Antipoden eingesetzt werden.

Gemische von Racematen lassen sich nach an sich bekannten Methoden, insbesondere mit Hilfe von physikalischen Trennverfahren,

wie fraktionierte Adsorption und Elution, inkl. Chromatographie, fraktioniertes Kristallisieren und Destillieren, etc., in die Racemate auftrennen.

Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer mit der racemischen Verbindung ein Salz bildenden optisch aktiven Verbindung, insbesondere einer entsprechenden Säure, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchlich als optisch aktive Säuren sind z.B. die D- und L-Formen von Weinsäure, Bis-O,O'-(p-toluoyl)-weinsäure, Aepfelsäure, Mandelsäure, Camphersulfonsäure, Glutaminsäure, Asparaginsäure oder Chinasäure. Vorteilhafterweise isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen die Reaktionskomponenten gegebenenfalls in Form ihrer Salze vorliegen.

Zweckmässig verwendet man für die Durchführung der erfindungsgemässen Reaktionen solche Ausgangsstoffe, die zu den eingangs besonders erwähnten Gruppen von Endstoffen und zu den, z.B. in den Beispielen, beschriebenen oder hervorgehobenen Endstoffen führen.

Die Ausgangsstoffe sind bekannt oder können, falls sie neu sind, nach an sich bekannten Methoden, wie oben, z.B. analog wie

in den Beispielen beschrieben, erhalten werden. Neue Ausgangsstoffe bilden ebenfalls einen Gegenstand der Erfindung. Die Erfindung betrifft auch verfahrensgemäss erhältliche Zwischenprodukte.

Die neuen Verbindungen können z.B. in Form pharmazeutischer Präparate Verwendung finden, welche eine pharmakologisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und/oder Schmiermitteln, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen, oder Adsorptionsmittel, Farbstoffe, Geschmackstoffe und Süssmittel aufweisen. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten etwa 0,1 % bis 100 %, insbesondere von etwa 1 % bis etwa 50 %, Lyophilisate bis zu 100 % des Aktivstoffes.

Die neuen Verbindungen der allgemeinen Formel I sowie ihre pharmazeutisch annehmbaren Säureadditionssalze werden vorzugsweise peroral verabreicht. Die täglichen Dosen bewegen sich zwischen 0,5 und 30 mg/kg für Mammalien und liegen für solche von ca. 70 kg Gewicht je nach individuellem Zustand und Alter vorzugsweise zwischen 50 und 1000 mg, insbesondere zwischen 100 und 500 mg. Entsprechende orale Doseneinheitsformen, z.B. Dragées oder Tabletten oder Kapseln, enthalten vorzugsweise 25 bis 500 mg, insbesondere 50 bis 250 mg eines erfindungsgemässen Wirkstoffes, d.h. einer Verbindung der allgemeinen Formel I oder eines pharmazeutisch annehmbaren Säureadditionssalzes einer solchen, zusammen mit pharmazeutischen Trägerstoffen.

Die folgende Vorschrift soll die Herstellung von Tabletten näher erläutern:

a) 500 g 5-Propyl-5-(4-pyridyl)-2,3,5,6-tetrahydro-imidazo-[1,2-c]chinazolin werden mit 500 g Lactose und 340 g Kartoffelstärke vermischt, die Mischung mit einer alkoholischen Lösung von 10 g Gelatine befeuchtet und durch ein Sieb granuliert. Nach dem Trocknen mischt man 60 g Kartoffelstärke, 60 g Talk, 10 g Magnesiumstearat und 20 g hochdisperses Siliciumdioxid zu und presst die Mischung zu 10'000 Tabletten von je 150 mg Gewicht und 50 mg Wirkstoffgehalt, die gewünschtenfalls mit Teilkerben zur feineren Anpassung der Dosierung versehen sein können.

Anstelle des vorgenannten Wirkstoffes kann man z.B. auch 500,0 g von 5-Phenyl-5-(4-pyridyl)-3,4,6,7-tetrahydro-2H-pyrimido[1,2-c]-chinazolin, von dessen Methansulfonat, von dessen Acetat-dihydrat oder

von einem anderen pharmazeutisch annehmbaren Säureadditionssalz desselben verwenden.

Die nachfolgenden Beispiele erläutern die Herstellung der neuen Verbindungen der allgemeinen Formel I sowie von bisher nicht bekannten Ausgangsstoffen näher, sollen jedoch den Umfang der Erfindung in keiner Weise beschränken. Die Temperaturen sind in Celsiusgraden angegeben.

Beispiel 1: Zu einer Lösung von 8,06 g (0,05 Mol) 2-(2-Aminophenyl)-4,5-dihydro-1H-imidazol (vgl. US-PS 3 920 687) in 125 ml 1,8-n. methanolischer Chlorwasserstofflösung gibt man bei 0° 12,11 g (0,1 Mol) Methyl-(4-pyridyl)-keton [vgl. J.Med.Chem. 14, 551 (1971)]. Das Reaktionsgemisch wird 60 Stunden bei Raumtemperatur gerührt und anschliessend im Vakuum zur Trockne eingedampft. Man versetzt den Rückstand mit 125 ml 1-n. Natronlauge und extrahiert mehrmals mit Chloroform. Die vereinigten,mit Wasser neutral gewaschenen Chloroformphasen werden nach Trocknen über Natriumsulfat eingedampft und der Rückstand aus Isopropanol umkristallisiert. Das erhaltene 5-Methyl-5-(4-pyridyl)-2,3,5,6-tetrahydro-imidazo[1,2-c]chinazolin schmilzt bei 227-228°.

Analog erhält man unter Verwendung von 12,11 g (0,1 Mol) Methyl-(3-pyridyl)-keton [vgl. Liebigs Ann. Chem. 486, 95 (1931)] 5-Methyl-5-(3-pyridyl)-2,3,5,6-tetrahydro-imidazo[1,2-c]chinazolin, Smp. 170 - 171° (aus Aethanol-Aether);

und unter Verwendung von 12,11 g (0,1 Mol) Methyl-(2-pyridyl)-keton [vgl. J.Med.Chem. 14, 551 (1971)] das 5-Methyl-5-(2-pyridyl)-2,3,5,6-tetrahydro-imidazo[1,2-c]chinazolin, Smp. 202-203° (aus Aethanol-Aether).

Der Ausgangsstoff 2-(2-Aminophenyl)-4,5-dihydro-1H-imdaizol kann, einfacher als in der US-PS 3 920 687 beschrieben, auch wie folgt hergestellt werden:

a) Ein Gemisch von 118,14 g (1 Mol) 2-Amino-benzonitril, 120,2 g (2 Mol) Aethylendiamin und 0,5 ml Schwefelkohlenstoff wird während 16 Stunden unter Rühren und Einleiten von Stickstoff auf 150°C erhitzt, wobei Ammoniak entwickelt wird. Dann dampft man das Reaktionsgemisch im Vakuum ein, nimmt den Rückstand unter Zusatz von Aktivkohle in heissem Aethylacetat auf, filtriert und versetzt das Filtrat unter starkem Rühren mit Hexan. Beim Abkühlen kristallisiert das 2-(2-Aminophenyl)-4,5-dihydro-1H-imidazol aus, Smp. 60-64°. Nach Umkristallisation aus Aether/Hexan schmilzt das Produkt bei 63-64°.

Beispiel 2: Eine Lösung von 8,06 g (0,05 Mol) 2-(2-Aminophenyl)-4,5-dihydro-1H-imidazol, 12,11 g (0,1 Mol) Methyl-(4-pyridyl)-keton und 0,8 g (0,0042 Mol) p-Toluolsulfonsäure-monohydrat in 200 ml Toluol wird 15 Stunden unter Wasserabscheidung zum Kochen unter Rückfluss erhitzt. Man kühlt, filtriert den gebildeten Niederschlag ab und verteilt diesen zwischen 1-n. Natronlauge und Methylenchlorid. Die mit Wasser neutral gewaschene und über Natriumsulfat getrocknete Methylenchlorid-Phase wird eingedampft. Das nach Umkristallisation des Rückstands aus Isopropanol erhaltene 5-Methyl-5-(4-pyridyl)-2,3,5,6-tetrahydro-imidazo[1,2-c]chinazolin schmilzt bei 228 - 229°.

Beispiel 3: Analog Beispiel 1 erhält man ausgehend von 8,06 g (0,05 Mol) 2-(2-Aminophenyl)-4,5-dihydro-1H-imidazol, 12,68 g (0,085 Mol) Propyl-(4-pyridyl)-keton [vgl. J. Chem. Soc. (C) 1969, 2134] und 200 ml 1,8-n. methanolischer Chlorwasserstofflösung das 5-Propyl-5-(4-pyridyl)-2,3,5,6-tetrahydro-imidazo[1,2-c]chinazolin, Smp. 241 - 242°(aus Isopropanol-Aethylacetat).

Beispiel 4: Analog Beispiel 1 erhält man ausgehend von 8,06 g (0,05 Mol) 2-(2-Aminophenyl)-4,5-dihydro-1H-imidazol, 8,81 g (0,08 Mol) (2-Furyl)-methylketon [vgl. Helv.Chim.Acta 13, 356 (1930)] und 150 ml 1,8-n. methanolischer Chlorwasserstofflösung das 5-Methyl-5-(2-furyl)-2,3,-5,6-tetrahydro-imidazo[1,2-c]chinazolin, Smp. 195 - 196° (aus Aethanol).

Beispiel 5: Analog Beispiel 1 werden 8,06 g (0,05 Mol) 2-(2-Aminophenyl)-4,5-dihydro-1H-imidazol mit 20,22 g (0,1 Mol) 3-Oxoglutarsäure-diäthylester in 240 ml 1,8-n. methanolischer Chlorwasserstofflösung umgesetzt. Das erhaltene, rohe 5,5-Bis-(äthoxycarbonylmethyl)-2,3,5,6-tetrahydroimidazo[1,2-c]chinazolin wird in 2,1-n. äthanolischer Salzsäure gelöst. Durch Zugabe von Aether wird das Hydrochlorid ausgefällt. Es schmilzt bei 213 - 214°.

Beispiel 6: Zu einer Lösung von 9,78 g (0,05 Mol) 2-(2-Amino-4-chlorphenyl)-4,5-dihydro-1H-imidazol (vgl. US-PS 3922 282) in 160 ml 2,5-n. methanolischer Chlorwasserstofflösung gibt man bei 0° 12,11 g (0,1 Mol) Methyl-(4-pyridyl)-keton. Das Reaktionsgemisch wird 15 Stunden unter Rückfluss gekocht und anschliessend im Vakuum eingeengt. Nach Zugabe von Aether bildet sich ein Niederschlag von Mono- oder Dihydrochlorid von 5-Methyl-5-(4-pyridyl)-8-chlor-2,3,5,6-tetrahydro-imidazo[1,2-c]chinazolin, den man abfiltriert und zwischen 1-n. Natronlauge und Chloroform verteilt. Die organische Phase wird mit Wasser neutral gewaschen, über Natriumsulfat getrocknet und eingedampft. Durch Umkristallisation des Rückstands aus Acetonitril/Isopropanol erhält man das 5-Methyl-5-(4-pyridyl)-8-chlor-2,3,5,6-tetrahydro-imidazo[1,2-c]chinazolin, Smp. 242 - 244°.

Analog erhält man unter Verwendung von 12,11 g (0,1 Mol) Methyl-(2-pyridyl)-keton das 5-Methyl-5-(2-pyridyl)-8-chlor-2,3,5,6-tetrahydro-imidazo[1,2-c]chinazolin, Smp. 247 - 249° (aus Aethanol).

Beispiel 7: 8,06 g (0,05 Mol) 2-(2-Aminophenyl)-4,5-dihydro-1H-imidazol und 10,63 g (0,055 Mol) β-Oxo-4-pyridinpropionsäureäthylester [vgl. J.Am.Chem. Soc. 67, 1468 (1945)] werden in 80 ml 2,1-n. äthanolischer Chlorwasserstofflösung 15 Stunden bei 90° im geschlossenen Gefäss erhitzt. Das Reaktionsgemisch wird zur Trockne eingedampft und der Rückstand zwischen 2-n. Natronlauge und Chloroform verteilt. Die mit Wasser neutral gewaschene und über Natriumsulfat getrocknete organische Phase wird eingedampft. Den Rückstand filtriert

man mit einem Gemisch Chloroform: Methanol: konz. Ammoniak = 150 : 50 : 1 über Kieselgel der Korngrösse 0,063 - 0,200 mm. Die das gewünschte Produkt enthaltenden Fraktionen werden eingedampft und der Rückstand aus Isopropanol umkristallisiert. Der erhaltene 5-(4-Pyridyl)-2,3,5,6-tetrahydro-imidazo[1,2-c]chinazolin-5-essigsäure-äthylester schmilzt bei 190 - 192°. Bei der obigen Filtration durch Kieselgel wird als Nebenprodukt 5-Methyl-5-(4-pyridyl)-2,3,5,6-tetrahydro-imidazo[1,2-c]chinazolin, Smp. 226 - 228° (aus Isopropanol) erhalten.

Analog kann der 5-(4-Pyridyl)-2,3,5,6-tetrahydro-imidazo[1,2-c]-chinazolin-5-essigsäureäthylester auch erhalten werden, wenn man bei der Reaktion anstelle von 2-(2-Aminophenyl)-4,5-dihydro-1H-imidazol ein entsprechendes Salz, z.B. das 2-(2-Aminophenyl)-4,5-dihydro-1H-imidazol-dihydrochlorid-hydrat (J.Med.Chem. 13, 697-704 (1970)) verwendet.

Beispiel 8: Zu einer Lösung von 8,06 g (0,05 Mol) 2-(2-Aminophenyl)-4,5-dihydro-1H-imidazol in 125 ml 1,8-n. methanolischer Chlorwasserstofflösung gibt man bei 0° 18,22 g (0,1 Mol) Benzophenon. Das Reaktionsgemisch wird 15 Stunden bei Raumtemperatur gerührt. Man gibt Aether zu und filtriert den entstandenen Niederschlag des Hydrochlorids von 5,5-Diphenyl-2,3,5,6-tetrahydro-imidazo[1,2-c]chinazolin ab. Dieses wird noch zweimal aus Aethanol/Aether umkristallisiert. Es schmilzt oberhalb 300°.

Analog erhält man unter Verwendung von 5,8 g (0,1 Mol) Aceton das 5,5-Dimethyl-2,3,5,6-tetrahydro-imidazo[1,2-c]chinazolin-hydrochlorid, Smp. 249 - 250° (aus Aethanol-Aether).

Beispiel 9: Analog Beispiel 8 werden 8,06 g (0,05 Mol) 2-(2-Aminophenyl)-4,5-dihydro-1H-imidazol und 8,10 g (0,06 Mol) Methyl-[(2-pyridyl)-methyl]-keton [vgl. Helv.Chim.Acta 45, 729 (1962)]

in 125 ml 1,8-n. methanolischer Chlorwasserstofflösung umgesetzt. Das Reaktionsgemisch wird jedoch eingedampft und der Rückstand aus Aethanol/Aether und Chloroform/Methanol/Aether umkristallisiert. Das Dihydrochlorid von 5-Methyl-5-[(2-Pyridyl)-methyl]-2,3,5,6-tetrahydro-imidazo[1,2-c]chinazolin beginnt sich bei etwa 150° zu zersetzen.

Beispiel 10: Analog Beispiel 8 werden 8,06 g (0,05 Mol) 2-(2-Aminophenyl)-4,5-dihydro-1H-imidazol und 6,75 g (0,05 Mol) Methyl-(6-methyl-2-pyridyl)-keton [vgl. J. Med. Pharm. Chem. 3, 561 (1961)] in 125 ml 1,8-n. methanolischer Chlorwasserstofflösung umgesetzt. Das Reaktionsgemisch wird jedoch eingedampft und der Rückstand aus Wasser und Aethanol/Aether umkristallisiert. Das Hydrochlorid von 5 Methyl-5-(6-methyl-2-pyridyl)-2,3,5,6-tetrahydro-imidazo[1,2-c]chinazolin schmilzt bei 269 - 270°.

Beispiel 11: Analog Beispiel 8 werden 8,06 g (0,05 Mol) 2-(2-Aminophenyl)-4,5-dihydro-1H-imidazol und 10,13 g (0,055 Mol) Di-(2-pyridyl)-keton [vgl. Rec. trav. chim. 70, 1054 (1951)] in 240 ml 1,8-n. methanolischer Chlorwasserstofflösung umgesetzt. Das Reaktionsgemisch wird jedoch eingedampft, der Rückstand in Wasser gelöst und die Lösung mit 2-n. Natronlauge auf pH 8 gestellt. Der gebildete Niederschlag wird abfiltriert und aus Aethanol/Aether umkristallisiert. Das erhaltene Hydrochlorid von 5,5-Di-(2-pyridyl)-2,3,5,6-tetrahydro-imidazo[1,2-c]chinazolin schmilzt oberhalb 300°.

Beispiel 12: Zu einer konzentrierten, schwach siedenden äthanolischen Lösung von 5,28 g (0,02 Mol) 5-Methyl-5-(4-pyridyl)-2,3,5,6-tetrahydro-imidazo[1,2-c]chinazolin gibt man in der Siedehitze eine ebenfalls schwach siedende, konzentrierte Lösung von 2,32 g (0,02 Mol) Maleinsäure. Nach dem Abkühlen wird das gebildete Maleat abfiltriert und aus Isopropanol umkristallisiert, worauf es bei 170 - 171° schmilzt.

Analog erhält man unter Verwendung von 2,32 g (0,02 Mol) Fumarsäure das entsprechende Fumarat. Dieses schmilzt nach Umkristallisation aus Methanol bei 237 - 239°.

Beispiel 13: Analog Beispiel 1 werden 8,06 g (0,05 Mol) 2-(2-Aminophenyl)-4,5-dihydro-1H-imidazol und 12,24 g (0,075 Mol) Butyl-(4-pyridyl)-keton [vgl. J.Chem. Soc. (C) 1969, 2134] in 150 ml 2,7-n. methanolischer Chlorwasserstofflösung umgesetzt. Bei der Aufarbeitung wird anstelle von 1-n. Natronlauge verdünnte wässrige Ammoniaklösung verwendet. Das erhaltene 5-Butyl-5-(4-pyridyl)-2,3,5,6-tetrahydro-imidazo[1,2-c]chinazolin schmilzt nach Umkristallisation aus Aethylacetat bei 257 - 259°.

Analog erhält man unter Verwendung von 15,40 g (0,075 Mol) Heptyl-(4-pyridyl)-keton [vgl. J.Med.Chem. 14, 551 (1971)] das 5-Heptyl-5-(4-pyridyl)-2,3,5,6-tetrahydro-imidazo-[1,2-c]chinazolin, Smp. 161 - 163° (aus Aceton/Petroläther), und unter Verwendung von 15,85 g (0,075 Mol) Phenäthyl-(4-pyridyl)-keton [vgl. J. Chem. Soc. (C) 1969, 2134] das 5-Phenäthyl-5-(4-pyridyl)-2,3,5,6-tetrahydro-imidazo[1,2-c]chinazolin, Smp. 187 - 188° (aus Aethylacetat.

Beispiel 14: Analog Beispiel 13 werden 6,44 g (0,04 Mol) 2-(2-Aminophenyl-4,5-dihydro-1H-imidazol und 11,0 g (0,06 Mol) Phenyl-(4-pyridyl)-keton umgesetzt, wobei jedoch das Reaktionsgemisch 40 Stunden bei Raumtemperatur gerührt und anschliessend 2 Stunden unter Rückfluss gekocht wird. Das erhaltene 5-Phenyl-5-(4-pyridyl)-2,3,5,6-tetrahydro-imidazo[1,2-c]chinazolin schmilzt nach Umkristallisation aus Chloroform-Aether und hierauf aus Aethylacetat bei 198 - 200°.

Beispiel 15: Analog Beispiel 1, jedoch unter Einhaltung einer Reaktionsdauer von 15 Stunden, erhält man ausgehend von 3,91 g (0,02 Mol) 2-(2-Amino-5-chlorphenyl)-4,5-dihydro-1H-imidazol, 4,47 g (0,03 Mol) Propyl-(4-pyridyl)-keton und 50 ml 2-n. methanolischer Chlorwasserstofflösung das 5-Propyl-5-(4-pyridyl)-9-chlor-2,3,5,6-tetrahydro-imidazo[1,2-c]chinazolin, Smp. 181,5 - 183° nach Umkristallisation aus Chloroform-Aether und hierauf aus Aethylacetat.

Das 2-(2-Amino-5-chlorphenyl)-4,5-dihydro-1H-imidazol erhält man wie folgt:

a) Eine Lösung von 15,26 g (0,1 Mol) 2-Amino-5-chlor-benzonitril, 6,0 g (0,1 Mol) 1,2-Aethandiamin und 20 Tropfen Schwefelkohlenstoff in 100 ml Aethanol wird 22 Stunden unter Rückfluss gekocht. Nach erneuter Zugabe von 3,0 g (0,05 Mol) 1,2-Aethandiamin und 20 Tropfen Schwefelkohlenstoff kocht man das Gemisch nochmals 50 Stunden unter Rückfluss, dampft dann das Reaktionsgemisch im Vakuum ein und kristallisiert den Rückstand aus Methylenchlorid-Hexan um. Das erhaltene 2-(2-Amino-5-chlorphenyl)-4,5-dihydro-1H-imidazol schmilzt bei 105 - 107°.

Beispiel 16: Analog Beispiel 15 erhält man ausgehend von 8,05 g (0,05 Mol) 2-(2-Aminophenyl)-4,5-dihydro-1H-imidazol, 8,15 g (0,055 Mol) Phenyl-propyl-keton und 150 ml 2,5-n. methanolischer Chlorwasserstofflösung das 5-Phenyl-5-propyl-2,3,5,6-tetrahydro-imidazo[1,2-c]chinazolin, Smp. 247 - 249° (aus Methanol-Aether).

Beispiel 17: Analog Beispiel 1, jedoch unter Kochen unter Rückfluss während 22 Stunden, erhält man ausgehend von 9,46 g (0,05 Mol) 2-(2-Amino-4,6-dimethylphenyl)-4,5-dihydro-1H-imidazol, 11,93 g (0,08 Mol) Propyl-(4-pyridyl)-keton und 165 ml 2,5-n. methanolischer Chlorwasserstofflösung das 5-Propyl-5-(4-pyridyl)-8,10-dimethyl-2,3,5,6-tetrahydro-imidazo[1,2-c]chinazolin, Smp. 187 - 189° nach Umkristallisation aus Aether und aus Aethylacetat-Hexan. Weiteres Reaktionsprodukt kann man erhalten, wenn man die Mutterlauge nach der ersten Umkristallisation aus Aether eindampft und den Rückstand, der noch Ausgangsmaterialien enthält, nochmals mit 80 ml 2,5-n. methanolischer Chlorwasserstofflösung 15 Stunden unter Rückfluss kocht.

Das 2-(2-Amino-4,6-dimethylphenyl)-4,5-dihydro-1H-imidazol erhält man wie folgt:

a) Ein Gemisch aus 21,93 g (0,15 Mol) 2-Amino-4,6-dimethylbenzonitril [vgl. Polish Journal of Chemistry 52, 1389 (1978)] und 34,85 g (0,15 Mol) 2-Aminoäthyl-ammonium-toluol-4-sulfonat [vgl. J.Chem.Soc., 497 (1947)] wird während 3 Stunden unter Rühren bei einer Badtemperatur von 250° erhitzt, wobei aus der Schmelze Ammoniak freigesetzt wird. Man gibt noch einmal 20 g (0,086 Mol) 2-Aminoäthyl-ammonium-toluol-4-sulfonat zu und erhitzt weitere 3 1/2 Stunden bei einer Badtemperatur von 250 - 260°. Nach dem Abkühlen wird das Reaktionsgemisch zwischen Chloroform und 10 %iger Natronlauge verteilt, die organische Phase mit Wasser gewaschen, über Natriumsulfat getrocknet und eingedampft. Nach dem Versetzen des Rückstands mit Aether kristallisiert das rohe 2-(2-Amino-4,6-dimethylphenyl)-4,5-dihydro-1H-imidazol aus, das nach Umkristallisation aus Aethylacetat bei 141 - 142° schmilzt.

Beispiel 18: 9,46 (0,05 Mol) 2-(2-Amino-4,6-dimethylphenyl)-4,5-dihydro-1H-imidazol [vgl. Beispiel 17a) und 13,74 g (0,075 Mol) Phenyl-(4-pyridyl)-keton werden in 200 ml 2,5-n. methanolischer Chlorwasserstofflösung 22 Stunden unter Rückfluss gekocht. Nach Aufarbeitung analog Beispiel 7 schmilzt das erhaltene 5-Phenyl-5-(4-pyridyl)-8,10-dimethyl-2,3,5,6-tetrahydro-imidazo[1,2-c]-chinazolin bei 249 - 251° (aus Isopropanol-Aether).

Beispiel 19: Zu einer Suspension von 1,32 g (0,005 Mol) 5-Methyl-5-(4-pyridyl)-2,3,5,6-tetrahydro-imidazo[1,2-c]chinazolin in 13 ml Dimethylformamid gibt man 0,28 g (0,0064 Mol) Natriumhydrid-Dispersion (55 % in Oel) und rührt das Gemisch eine Stunde bei 70°. Nach dem Abkühlen auf Raumtemperatur werden 0,91 g (0,0064 Mol) Methyljodid zugetropft. Man rührt das Gemisch noch 10 Minuten weiter, kühlt es dann auf 0°, tropft 2 ml Wasser in das Reaktionsgemisch, dampft es im Vakuum ein und verteilt den Rückstand zwischen Aethylacetat und Wasser. Die organische Phase wird mit Wasser gewaschen, über Magnesiumsulfat getrocknet und eingedampft. Durch Umkristallisation des Rückstands aus Aethylacetat-Petroläther erhält man das reine 5,6-Dimethyl-5-(4-pyridyl)-2,3,5,6-tetrahydro-imidazo[1,2-c]-chinazolin, Smp. 153 - 154°.

Beispiel 20: Analog Beispiel 1, jedoch unter Kochen unter Rückfluss während 7 Stunden, erhält man unter Verwendung von 9,46 g (0,05 Mol) 2-(2-Aminophenyl)-4,4(oder 5,5)-dimethyl-4,5-dihydro-1H-imidazol (vgl. US. 3894040 und US 3920687), 9,08 g (0,075 Mol) Methyl-(4-pyridyl)-keton und 160 ml 2,5-n. methanolischer Chlorwasserstofflösung das 2,2,5-Trimethyl-5-(4-pyridyl)-2,3,5,6-tetrahydro-imidazo[1,2-c]chinazolin, Smp. 138 - 141° (aus Aethylacetat-Hexan).

Beispiel 21: 15,92 g (0,1 Mol) 2-(2-Aminophenyl)-1H-imidazol [vgl. Il Farmaco, Ed. Sc. 30, 536 (1975)] und 20,89 g (0,14 Mol) Propyl-(4-pyridyl)-keton werden in 240 ml 2,5-n. methanolischer Chlorwasserstofflösung während 15 Stunden bei 20° gerührt und anschliessend noch 12 Stunden unter Rückfluss gekocht. Man dampft dann das Reaktionsgemisch zur Trockene ein und verteilt den Rückstand zwischen 2-n. Natronlauge und Chloroform. Die organische Phase wird mit Wasser gewaschen, über Natriumsulfat getrocknet und eingedampft. Das nach Umkristallisation des Rückstands aus Aethylacetat erhaltene 5-Propyl-5-(4-pyridyl)-5,6-dihydro-imidazo[1,2-c]-chinazolin schmilzt bei 199 - 200°.

Beispiel 22: Analog Beispiel 1, jedoch unter Einhaltung einer Reaktionsdauer von 20 Stunden bei 20°, erhält man ausgehend von 3,18 g (0,02 Mol) 2-(2-Aminophenyl)-1H-imidazol, 3,63 g (0,03 Mol) Methyl-(4-pyridyl)-keton und 50 ml 3-n. methanolischer Chlorwasserstofflösung das 5-Methyl-5-(4-pyridyl)-5,6-dihydro-imidazo[1,2-c]-chinazolin, Smp. 208 - 210° (aus Chloroform-Aether).

Beispiel 23: Eine Lösung von 8,76 g (0,05 Mol) 2-(2-Aminophenyl)-1,4,5,6-tetrahydro-pyrimidin [vgl. J. Heterocyclic Chem. 15, 877 (1978)] und 13,74 g (0,075 Mol) Phenyl-(2-pyridyl)-keton in 125 ml 2,5-n. methanolischer Chlorwasserstofflösung wird 48 Stunden unter Rückfluss gekocht und anschliessend eingedampft. Man verteilt den Rückstand zwischen 2-n. Natronlauge und Chloroform, wäscht die organische Phase mit Wasser und dampft diese nach Trocknen über Natriumsulfat am Vakuum ein. Der ölige Rückstand kristallisiert beim Verrühren mit Aether. Nach Umkristallisation aus Aethanol-Hexan schmilzt das erhaltene 6-Phenyl-6-(2-pyridyl)-3,4,6,7-tetrahydro-2H-pyrimido[1,2-c]chinazolin bei 218 - 219°.

Analog erhält man:

Unter Verwendung von 17,1 g (0,075 Mol) (3-Nitrophenyl)-(4-pyridyl)-keton [vgl. Monatshefte für Chemie 107, 1449 (1976)] das 6-(3-Nitrophenyl)-6-(4-pyridyl)-3,4,6,7-tetrahydro-2H-pyrimido[1,2-c]chinazolin, Smp. 115 - 117° (aus Aethylacetat, die Verbindung enthält ein Mol Aethylacetat);

unter Verwendung von 13,81 g (0,075 Mol) 2,2'-Dipyridylketon - jedoch unter Kochen unter Rückfluss in 150 ml 3-n. methanolischer Chlorwasserstofflösung während 20 Stunden, das 6,6-Di-(2-pyridyl)-3,4,6,7-tetrahydro-2H-pyrimido[1,2-c]chinazolin, Smp. 265 - 266° (aus Methanol);

unter Verwendung von 11,12 g (0,075 Mol) Phenyl-propyl-keton - jedoch unter Erhitzen im Bombenrohr bei 150° während 4 Stunden - das 6-Phenyl-6-propyl-3,4,6,7-tetrahydro-2H-pyrimido[1,2-c]-chinazolin, Smp. 190 - 192° (aus Aethylacetat);

und durch Reaktion von 8,76 g (0,05 Mol) 2-(2-Aminophenyl)-1,4,5,6-tetrahydro-pyrimidin mit 8,7 g (0,15 Mol) Aceton in 100 ml 2,5-n. methanolischer Chlorwasserstofflösung während 15 Stunden bei 20° das 6,6-Dimethyl-3,4,6,7-tetrahydro-2H-pyrimido[1,2-c]-chinazolin, Smp. 218 - 220° (aus Aethylacetat).

Die Ausgangsverbindung, das 2-(2-Aminophenyl)-1,4,5,6-tetrahydro-pyrimidin kann auch folgendermassen erhalten werden:

a) Ein Gemisch von 50 g (0,42 Mol) 2-Amino-benzonitril, 62,27 g (0,84 Mol) 1,3-Propandiamin und 5 Tropfen Schwefelkohlenstoff wird während 24 Stunden unter Rühren und Einleiten von Stickstoff auf 150° erhitzt, wobei Ammoniak entwickelt wird. Man dampft dann das Reaktionsgemisch im Vakuum ein und nimmt den Rückstand in Aether auf. Das 2-(2-Aminophenyl)-1,4,5,6-tetrahydro-pyrimidin kristallisiert dabei aus der ätherischen Lösung aus, Smp. 99 - 101°.

Beispiel 24: Zu einer Lösung von 34,0 g (0,194 Mol) 2-(2-Aminophenyl)-1,4,5,6-tetrahydro-pyrimidin und 53,3 g (0,291 Mol) Phenyl-(4-pyridyl)-keton in 300 ml 3-n. methanolischer Chlorwasserstofflösung gibt man 20 g Molekularsieb ($3.10^{-10}$ m, Perlform etwa 2 mm; Merck) und kocht das Reaktionsgemisch 16 Stunden unter Rückfluss. Das Molekularsieb wird abfiltriert, das Filtrat eingedampft und der Rückstand analog Beispiel 23 aufgearbeitet. Das erhaltene 6-Phenyl-6-(4-pyridyl)-3,4,6,7-tetrahydro-2H-pyrimido[1,2-c]chinazolin schmilzt bei 243 - 245° (aus Isopropanol-Aether).

Analog kann das 6-Phenyl-6-(4-pyridyl)-2,4,6,7-tetrahydro-2H-pyrimido[1,2-c]chinazolin auch erhalten werden, wenn man bei der Reaktion anstelle von 2-(2-Aminophenyl)-1,4,5,6-tetrahydro-pyrimidin ein entsprechendes Salz, z.B. das 2-(2-Aminophenyl)-1,4,5,6-tetrahydro-pyrimidin-dihydrochlorid (vgl. J.Med.Chem. 13, 697-704 (1970), oder anstelle des freien Ketons dessen Imin verwendet.

Beispiel 25: Eine Lösung von 13,15 g (0,075 Mol) 2-(2-Aminophenyl)-1,4,5,6-tetrahydro-pyrimidin und 10,41 g (0,086 Mol) Methyl-(4-pyridyl)-keton in 200 ml 1,83-n. methanolischer Chlorwasserstofflösung wird während 2 1/2 Tagen bei 20° gerührt. Anschliessend engt man das Reaktionsgemisch auf etwa die Hälfte des ursprünglichen Volumens ein, gibt Aether zu, filtriert den gebildeten Niederschlag ab und verteilt diesen zwischen Chloroform und 1-n. Natronlauge. Die mit Wasser gewaschene organische Phase wird über Natriumsulfat getrocknet, eingedampft und der Rückstand aus Isopropanol umkristallisiert. Das erhaltene 6-Methyl-6-(4-pyridyl)-3,4,6,7-tetrahydro-2H-pyrimido[1,2-c]chinazolin schmilzt bei 207 - 209°.

Beispiel 26: 8,76 g (0,05 Mol) 2-(2-Aminophenyl)-1,4,5,6-tetrahydro-pyrimidin und 13,74 g (0,075 Mol) Phenyl-(2-pyridyl)-keton werden in 90 g Polyphosphorsäure während 3 Stunden bei 150° gerührt. Man kühlt ab, versetzt das Reaktionsgemisch mit Eiswasser und einem Ueberschuss an wässriger Ammoniaklösung, extrahiert mit Chloroform und dampft die organische Phase nach Trocknen über Natriumsulfat im Vakuum ein. Der ölige Rückstand kristallisiert beim Verrühren mit Aether. Das erhaltene 6-Phenyl-6-(2-pyridyl)-3,4,6,7-tetrahydro-2H-pyrimido[1,2-c]chinazolin schmilzt bei 218 - 219° (aus Aethanol-Hexan).

Analog erhält man ausgehend von 13,74 g (0,075 Mol) Phenyl-(4-pyridyl)-keton - jedoch bei einer Reaktionsdauer von 2 Stunden bei 130° und 4 Stunden bei 150° sowie unter Verwendung von 140 g Polyphosphorsäure - das 6-Phenyl-6-(4-pyridyl)-3,4,6,7-tetrahydro-2H-pyrimido[1,2-c]chinazolin, Smp. 243 - 246° (aus Isopropanol-Aether).

Beispiel 27: Eine Lösung von 8,76 g (0,05 Mol) 2-(2-Aminophenyl)-1,4,5,6-tetrahydro-pyrimidin und 11,18 g (0,075 Mol) Propyl-(4-pyridyl)-keton in 200 ml 2-n. methanolischer Chlorwasserstofflösung wird 2 Tage unter Rückfluss gekocht und analog Beispiel 23 aufgearbeitet. Das erhaltene 6-Propyl-6-(4-pyridyl)-3,4,6,7-tetrahydro-2H-pyrimido[1,2-c]chinazolin schmilzt bei 221 - 223° (aus Aethanol-Aether).

Die bei der Umkristallisation erhaltene Mutterlauge, die noch Ausgangsmaterialien enthält, wird eingedampft und der Rückstand mit 60 g Polyphosphorsäure während einer Stunde bei 120° gerührt. Man kühlt ab, versetzt das Reaktionsgemisch mit Eiswasser und einem Ueberschuss an wässriger Ammoniaklösung, extrahiert mit Chloroform und dampft die organische Phase nach Trocknen über Natriumsulfat im Vakuum ein. Der Rückstand wird aus Aethanol-Aether umkristallisiert. Man erhält auf diese Weise eine zweite Charge 6-Propyl-6-(4-pyridyl)-3,4,6,7-tetrahydro-2H-pyrimido[1,2-c]chinazolin, Smp. 221 - 223°.

Analog erhält man unter Verwendung von 12,24 g (0,075 Mol) Butyl-(4-pyridyl)-keton das 6-Butyl-6-(4-pyridyl)-3,4,6,7-tetrahydro-2H-pyrimido[1,2-c]chinazolin, Smp. 197 - 199° (aus Chloroform-Hexan).

Beispiel 28: Ein Gemisch von 17,52 g (0,1 Mol) 2-(2-Aminophenyl)-1,4,5,6-tetrahydro-pyrimidin, 27,48 g (0,15 Mol) Phenyl-(4-pyridyl)-keton und 0,3 g Toluol-4-sulfonsäure-monohydrat wird während 24 Stunden bei 180° und einem Druck von etwa 270 mbar gerührt. Nach Abkühlen nimmt man die Schmelze in Aethylacetat auf. Dabei fällt rohes 6-Phenyl-6-(4-pyridyl)-3,4,6,7-tetrahydro-2H-pyrimido-[1,2-c]chinazolin aus, das nach Umkristallisation aus Isopropanol-Aether bei 243 - 245° schmilzt.

Beispiel 29: Unter Rühren und Einleiten von Stickstoff wird in eine Schmelze aus 100 g wasserfreiem Aluminiumchlorid und 50 g Natriumchlorid bei 160° ein Gemisch aus 20 g (0,114 Mol) 2-(2-Aminophenyl)-1,4,5,6-tetrahydro-pyrimidin und 31,2 g (0,170 Mol) Phenyl-(4-pyridyl)-keton eingetragen, wobei die Temperatur vorübergehend auf 205° steigt. Nach beendeter Zugabe rührt man das Reaktionsgemisch noch 20 Minuten bei 180° und lässt dann die Schmelze unter Abkühlen erstarren. Das Reaktionsgut wird in 700 ml heissem Wasser gelöst. Man wäscht mit Chloroform und versetzt die wässrige Phase mit 500 ml konzentrierter wässriger Ammoniaklösung. Die alkalische Phase wird mit 500 ml Chloroform während 3 Stunden gut gerührt und das Gemisch nach Zugabe von 300 g Kieselgur filtriert. Die Chloroformphase des Filtrats trocknet man über Natriumsulfat und dampft sie im Vakuum ein. Nach Umkristallisation des Rückstands aus Aethylacetat und aus Isopropanol-Aether schmilzt das erhaltene 6-Phenyl-6-(4-pyridyl)-3,4,6,7-tetrahydro-2H-pyrimido[1,2-c]-chinazolin bei 243 - 245°.

Beispiel 30: Zu einer Lösung von 6,8 g (0,02 Mol) 6-Phenyl-6-(4-pyridyl)-3,4,6,7-tetrahydro-2H-pyrimido[1,2-c]chinazolin in Methanol gibt man 1,92 g (0,02 Mol) Methansulfonsäure. Das Gemisch wird eingedampft und der Rückstand aus Aethanol-Aethylacetat umkristallisiert. Das erhaltene 6-Phenyl-6-(4-pyridyl)-3,4,6,7-tetrahydro-2H-pyrimido[1,2-c]chinazolin-methansulfonat schmilzt bei 249 - 250°.

Bei der Umkristallisation des obigen Methansulfonates aus Aethanol mit wenig Wasser und Zufügen von Hexan bis zur Trübung, Abfiltrieren und Trocknen erhält man je nach Trocknungsart das Methansulfonat-monohydrat bzw. das Methansulfonat-dihydrat, die beide bei 248-251° (mit Sintern ab 100°) schmelzen.

Beispiel 31: Analog Beispiel 23 erhält man ausgehend von 8,76 g (0,05 Mol) 2-(2-Aminophenyl)-1,4,5,6-tetrahydro-pyrimidin und 13,74 g (0,075 Mol) Phenyl-(3-pyridyl)-keton rohes 6-Phenyl-6-(3-pyridyl)-3,4,6,7-tetrahydro-2H-pyrimido[1,2-c]chinazolin, das man mit einem Gemisch Chloroform:Methanol:konz.Ammoniak = 40:10:1 über Kieselgel der Korngrösse 0,063 - 0,200 mm filtriert. Die das gewünschte Produkt enthaltenden Fraktionen werden eingedampft und der ölige Rückstand in Methanol gelöst. Zu dieser Lösung gibt man die methanolische Lösung einer äquimolaren Menge Fumarsäure. Nach Eindampfen im Vakuum wird der Rückstand aus Methanol-Aethylacetat umkristallisiert. Das erhaltene 6-Phenyl-6-(3-pyridyl)-3,4,6,7-tetrahydro-2H-pyrimido[1,2-c]chinazolin-fumarat enthält 1,5 Mol Fumarsäure und schmilzt bei 223 - 225°.

Beispiel 32: Analog Beispiel 25 erhält man ausgehend von 17,52 g (0,1 Mol) 2-(2-Aminophenyl)-1,4,5,6-tetrahydro-pyrimidin, 29,55 g (0,15 Mol) (4-Methylphenyl)-(4-pyridyl)-keton [vgl. Helv.Chim.Acta 52, 262 (1969)], 150 ml 2,5-n. methanolischer Chlorwasserstofflösung und 15 g Molekularsieb ($3.10^{-10}$m, Perlform etwa 2mm; Merck) rohes 6-(4-Methylphenyl)-6-(4-pyridyl)-3,4,6,7-tetrahydro-2H-pyrimido[1,2-c]-chinazolin, das man mit einem Gemisch von Chloroform:Methanol:konz. Ammoniak = 40:10:1 als Lösungs- und Eluiermittel an Kieselgel der Korngrösse 0,063 - 0,200 mm chromatographiert. Die das gewünschte Produkt enthaltenden einheitlichen Fraktionen werden eingedampft. Man löst den Rückstand in Aethanol und versetzt die äthanolische Lösung mit einer äquivalenten Menge Methansulfonsäure. Nach erneutem Eindampfen und Umkristallisation des Rückstands aus Aethanol-Hexan schmilzt das erhaltene 6-(4-Methylphenyl)-6-(4-pyridyl)-3,4,6,7-tetrahydro-2H-pyrimido[1,2-c]chinazolin-methansulfonat bei 257 - 259°.

Beispiel 33: Ein Gemisch von 17,52 g (0,1 Mol) 2-(2-Aminophenyl)-1,4,5,6-tetrahydro-pyrimidin, 18,32 g (0,1 Mol) Phenyl-(4-pyridyl)-keton, 500 ml Toluol und 70 g Kieselgel der Korngrösse 0,063 - 0,200 mm wird unter Rühren 18 Stunden am Wasserabscheider gekocht, dann

nocheinmal mit 20 g Kieselgel versetzt und weitere 15 Stunden am Wasserabscheider gekocht. Nach Filtration und Waschen des Filtergutes mit einem Chloroform-Methanolgemisch (1:1) dampft man das Filtrat im Vakuum ein und versetzt den Rückstand mit Aethylacetat, wobei 6-Phenyl-6-(4-pyridyl)-3,4,6,7-tetrahydro-2H-pyrimido[1,2-c]chinazolin ausfällt, das bei 234 - 237° schmilzt.

Beispiel 34: Analog Beispiel 19 werden 12,75 g (0,0375 Mol) 6-Phenyl-6-(4-pyridyl)-3,4,6,7-tetrahydro-2H-pyrimido[1,2-c]chinazolin, 2,19 g (0,05 Mol) Natriumhydrid-Dispersion (55 % in Oel) und 7,8 g (0,05 Mol) Aethyljodid in 110 ml Dimethylformamid umgesetzt. Man rührt jedoch 12 Stunden bei Raumtemperatur, tropft dann unter Kühlen 10 ml Wasser in das Reaktionsgemisch, dampft es im Vakuum ein und verteilt den Rückstand zwischen Chloroform und 1-n. Natronlauge. Die organische Phase wird mit Wasser gewaschen, über Natriumsulfat getrocknet und eingedampft. Den Rückstand chromotographiert man mit einem Gemisch von Chloroform:Methanol:konz. Ammoniak = 14:6:1 als Lösungs- und Eluiermittel an Kieselgel der Korngrösse 0,063 - 0,200 mm. Die das gewünschte Produkt enthaltenden, einheitlichen Fraktionen werden eingedampft und der Rückstand aus Aethanol-Hexan umkristallisiert.
Das erhaltene 6-Phenyl-6-(4-pyridyl)-7-äthyl-3,4,6,7-tetrahydro-2H-pyrimido[1,2-c]chinazolin schmilzt bei 230 - 231°. Das daraus mit Fumarsäure hergestellte Fumarat schmilzt bei 252 - 254° (aus Aethanol-Methanol 10:1).

Beispiel 35: Analog Beispiel 1, jedoch unter Kochen am Rückfluss während 5 Stunden, erhält man, ausgehend von 11,16 g (0,059 Mol) 2-(2-Aminophenyl)-4,4(oder 5,5)-dimethyl-4,5-dihydro-1H-imidazol, 13,27 g (0,089 Mol) Propyl-(4-pyridyl)-keton und 150 ml 3-n.methanolischer Chlorwasserstofflösung das 2,2-Dimethyl-5-propyl-5-(4-pyridyl)-2,3,5,6-tetrahydro-imidazo[1,2-c]-chinazolin, Smp. 224-226° nach Umkristallisation aus Aethylacetat und dann aus Aethanol-Aether.

Beispiel 36: Analog Beispiel 33 erhält man unter Verwendung von 16,32 g (0,1 Mol) 2-(2-Aminophenyl)-4,5-dihydro-1H-imidazol und 21,83 g (0,1 Mol) (4-Methoxyphenyl)-(2-thienyl)-keton, jedoch unter Kochen am Rückfluss während 7 Tagen und Chromatographie des Rohprodukts mit einem Gemisch Chloroform:Methanol = 9:1 über Kieselgel der Korngrösse 0,063-0,200 mm, das 5-(4-Methoxyphenyl)-5-(2-thienyl)-2,3,5,6-tetrahydro-imidazo(1,2-c]chinazolin, Smp. 207-208° (aus Aceton und Aethylacetat).

Beispiel 37: Zu einer Lösung von 8,06 g (0,05 Mol) 2-(2-Aminophenyl)-4,5-dihydro-1H-imidazol in 140 ml 2,6-n.methanolischer Chlorwasserstofflösung gibt man 11,68 g (0,05 Mol) 2-Chlor-5-acetyl-benzolsulfonamid [Arzneimittelforsch. 13, 269-280 (1963)] und rührt das Reaktionsgemisch 72 Stunden bei 20°. Der gebildete Niederschlag wird abfiltriert, in 360 ml Wasser suspendiert und die Suspension mit einem Ueberschuss 30%iger wässriger Ammoniaklösung 1/2 Stunde bei Raumtemperatur gerührt. Nach Filtration und Umkristallisation des Rückstandes aus Methanol schmilzt das erhaltene 5-(4-Chlor-3-sulfamoyl-phenyl)-5-methyl-2,3,5,6-tetrahydro-imidazo[1,2-c]chinazolin bei 274° (Zers.).

Beispiel 38: 17,52 g (0,1 Mol) 2-(2-Aminophenyl)-1,4,5,6-tetrahydropyrimidin, 21.32 g (0,1 Mol) 4-Methoxyphenyl-(4-pyridyl)-keton (J.Pharm.Sci. 62, 847-9 (1973) und 43,2 g (0,1Mol) Polyphosphatester (PPE, Fieser & Fieser, Reagents for Organic Synthesis, New York 1967, Seite 892) werden in 250 ml Chloroform während 96 Stunden unter Rückfluss gekocht. Das Reaktionsgemisch wird anschliessend mit einem Ueberschuss an 2-n. Natronlauge gerührt. Dann wird die organische Phase abgetrennt, mit wenig Wasser gewaschen, über Natriumsulfat getrocknet und eingedampft. Den Rückstand versetzt man mit Aethylenchlorid, wobei das gewünschte 6-(4-Methoxyphenyl)-6-(4-pyridyl)-3,4,6,7-tetrahydro-2H-pyrimido[1,2-c]chinazolin ausfällt. Es schmilzt bei 236-238° (aus Aethylenchlorid).

Beispiel 39: 17,52 g (0,1 Mol) 2-(2-Aminophenyl)-1,4,5,6-tetrahydropyrimidin, 18,32 g (0,1 Mol) Phenyl-(4-pyridyl)-keton und 43,2 g (0,1 Mol) Polyphosphatester werden in 150 ml Chloroform unter Rühren und schwachem Einleiten von Stickstoff während 45 Stunden unter Rückfluss gekocht. Nach Abkühlen und Zugabe von 250 ml 2-n. Natronlauge sowie 200 ml Chloroform zum Reaktionsgemisch rührt man diese noch 15 Minuten bei Raumtemperatur, trennt anschliessend die Chloroformphase ab und extrahiert die wässrige Phase noch zweimal mit je 150 ml Chloroform. Die vereinigten, mit wenig Wasser gewaschenen und über Natriumsulfat getrockneten organischen Phasen werden eingedampft und der ölige Rückstand mit 100 ml Aethylacetat gerührt, wobei das gewünschte 6-Phenyl-6-(4-pyridyl)-3,4,6,7-tetrahydro-2H-pyrimido-[1,2-c]chinazolin ausfällt, das nach Waschen mit Aethylacetat und Aether bei 237-239° schmilzt. Nach einer zusätzlichen Umkristallisation aus Aethanol/Hexan schmilzt das Produkt bei 240-242°.

Beispiel 40: 17,52 g (0,1 Mol) 2-(2-Aminophenyl)-1,4,5,6-tetrahydropyrimidin, 23,93 g (0,1 Mol) 4-(1,1-Dimethyläthyl)-phenyl-(4-pyridyl)-keton [J.Pharm.Sci. 62, 847-849 (1973)] und 43,2 g (0,1 Mol) Polyphosphatester werden in 250 ml Chloroform während 24 Stunden unter Rückfluss gekocht. Man kühlt das Reaktionsgemisch ab und gibt unter starkem Rühren 260 ml halbkonzentrierte (ca. 12,5% in Wasser) Ammoniaklösung zu. Nach 15 Minuten Rühren wird die Chloroformphase abgetrennt, über Natriumsulfat getrocknet und eingedampft. Man filtriert den Rückstand über Kieselgel der Korngrösse 0,063-0,200 mm mit einem Gemisch Chloroform:Methanol:konz.Ammoniak = 40:10:1 und dampft die das gewünschte Produkt enthaltenden Fraktionen ein. Nach Umkristallisation aus Acetonitril und Methylenchlorid-Hexan schmilzt das erhaltene 6-[4-(1,1-Dimethyläthyl)-phenyl]-6-[4-pyridyl]-3,4,6,7-tetrahydro-2H-pyrimido[1,2-c]chinazolin bei 142-145°.

Beispiel 41: 70 g Kieselgel H nach Stahl (z.B. der Firma E. Merck AG, Darmstadt) werden mit 600 ml Xylol versetzt und unter Rühren am Wasserabscheider so lange erhitzt, bis sich kein Wasser mehr abscheidet. Man kühlt ab, gibt dann 17,52 g (0,1 Mol) 2-(2-Aminophenyl)-1,4,5,6-tetrahydro-pyrimidin und 18,32 g (0,1 Mol) Phenyl-(4-pyridyl)-keton zu und kocht das Reaktionsgemisch 95 Stunden am Wasserabscheider. Nach Filtration und Waschen des Nutschrückstandes mit Aether wird das Filtergut mit Chloroform kontinuierlich extrahiert. Man dampft den Extrakt ein und versetzt den erhaltenen Rückstand mit Aethylacetat, wobei 6-Phenyl-6-(4-pyridyl)-3,4,6,7-tetrahydro-2H-pyrimido[1,2-c]chinazolin ausfällt, das bei 241-243° schmilzt.

Beispiel 42: Analog Beispiel 41, jedoch unter Verwendung von Toluol als Lösungsmittel und Erhitzen des Reaktionsgemisches während 40 Stunden, erhält man ausgehend von 17,52 g (0,1 Mol) 2-(2-Aminophenyl)-1,4,5,6-tetrahydro-pyrimidin und 21,77 g (0,1 Mol) (4-Chlorphenyl)-(4-pyridyl)-keton das 6-(4-Chlorphenyl)-6-(4-pyridyl)-3,4,6,7-tetrahydro-2H-pyrimido[1,2-c]chinazolin, welches nach Umkristallisation aus Aethylenchlorid und Acetonitril bei 215-217° schmilzt.

Beispiel 43: Analog Beispiel 41, jedoch unter Verwendung von Toluol und Erhitzen während 72 Stunden, werden 8,76 g (0,05 Mol) 2-(2-Aminophenyl)-1,4,5,6-tetrahydro-pyrimidin und 9,46 g (0,05 Mol) (4-Pyridyl)-(2-thienyl)-keton (J.Med.Chem. 12, 1093-6 (1969) umgesetzt. Nach Filtration und Waschen des Filterguts mit einem Lösungsmittelgemisch, Chloroform:Methanol = 1:1 wird das Filtrat eingedampft. Den Rückstand versetzt man mit Aethylacetat, wobei rohes 6-(4-Pyridyl)-6-(2-thienyl)-3,4,6,7-tetrahydro-2H-pyrimido[1,2-c]chinazolin ausfällt, das nach Umkristallisation aus Aethanol-Hexan bei 208-210° schmilzt.

Beispiel 44: Analog Beispiel 41 werden 17,52 g (0,1 Mol) 2-(2-Aminophenyl)-1,4,5,6-tetrahydro-pyrimidin und 19,92 g (0,1 Mol) (4-Hydroxyphenyl)-(4-pyridyl)-keton unter Verwendung von 500 ml Chlorbenzol als Lösungsmittel umgesetzt. Nach Filtration und Waschen des Kieselgels mit Aether wird das Filtergut dreimal mit je 300 ml eines Gemisches Chloroform:Methanol = 1:1 unter Erwärmen gerührt und dann filtriert. Man dampft die vereinigten Filtrate ein und reinigt den Rückstand durch Chromatographie über Kieselgel mit einem Gemisch Chloroform:Methanol:konz. Ammoniak = 40:10:1. Die das gewünschte Produkt enthaltenden Fraktionen werden vereinigt und eingedampft. Man kocht den kristallinen Rückstand in Methanol, kühlt ab und filtriert. Das erhaltene 6-(4-Hydroxyphenyl)-6-(4-pyridyl)-3,4,6,7-tetrahydro-2H-pyrimido[1,2-c]chinazolin-hemihydrat schmilzt bei 287-289° (Zers.).

Die Ausgangsverbindung, das (4-Hydroxyphenyl)-(4-pyridyl)-keton, wird wie folgt hergestellt:

Zu einer Lösung von 53,3 g (0,25 Mol) (4-Methoxyphenyl)-(4-pyridyl)-keton in 500 ml Methylenchlorid tropft man unter Rühren bei 5° eine Lösung von 187,9 g (0,75 Mol) Bortribromid in 750 ml Methylenchlorid. Das Reaktionsgemisch wird 15 Stunden bei Raumtemperatur gerührt und anschliessend mit einem Ueberschuss von 2-n. Natronlauge extrahiert. Man wäscht die alkalische Phase mit Chloroform und stellt sie anschliessend mit Eisessig sauer (pH ca. 5). Der gebildete Niederschlag wird abfiltriert und das Filtergut mit Isopropanol gewaschen. Nach Umkristallisation aus Dimethylformamid-Wasser schmilzt das erhaltene (4-Hydroxyphenyl)-(4-pyridyl)-keton bei 257-259°. (Vgl. Heterocycles 2, 423-426 (1974)).

Beispiel 45: Analog Beispiel 19 werden 12,75 g (0,0375 Mol) 6-Phenyl-6-(4-pyridyl)-3,4,6,7-tetrahydro-2H-pyrimido[1,2-c]chinazolin. 2,19 g (0,05 Mol) Natriumhydrid-Dispersion (55% in Oel) und 7,1 g (0,05 Mol) Methyljodid in 110 ml Dimethylformamid umgesetzt. Man rührt jedoch 12 Stunden bei Raumtemperatur, tropft dann unter Kühlen 10 ml Wasser in das Reaktionsgemisch, dampft es im Vakuum ein und verteilt den Rückstand zwischen Chloroform und 15%iger Natronlauge. Die mit Wasser gewaschene und über Natriumsulfat getrocknete organische Phase wird eingedampft und der Rückstand mit einem Gemisch Chloroform:Methanol: konz. Ammoniak = 40:10:1 über Kieselgel chromatographiert. Die das

gewünschte Produkt enthaltenden Fraktionen werden eingedampft und der Rückstand aus Chloroform-Aether umkristallisiert. Das erhaltene 6-Phenyl-6-(4-pyridyl)-7-methyl-3,4,6,7-tetrahydro-2H-pyrimido-[1,2-c]chinazolin schmilzt bei 183-184°.

Beispiel 46: Analog Beispiel 12 erhält man unter Verwendung äquivalenter Mengen von 5-Propyl-5-(4-pyridyl)-2,3,5,6-tetrahydro-imidazo-[1,2-c]chinazolin und äthanolischer Chlorwasserstofflösung das entsprechende Hydrochlorid, welches nach Umkristallisation aus Aethanol-Aether bei 278-280° schmilzt.

Analog erhält man bei Verwendung einer äquivalenten Menge Fumarsäure das 5-Propyl-5-(4-pyridyl)-2,3,5,6-tetrahydro-imidazo[1,2-c]chinazolin-fumarat, Smp. 215-217° (Zers.).

Beispiel 47: In 300 ml Aethanol löst man unter Erhitzen 34,04 g (0,1 Mol) 6-Phenyl-6-(4-pyridyl)-3,4,6,7-tetrahydro-2H-pyrimido[1,2-c]chinazolin und gibt dann unter Kühlen eine Lösung von 10,22 g 96%iger Schwefelsäure (0,1 Mol) in 19,5 g Wasser zu. Das beim Abkühlen ausgefallene Sulfat schmilzt nach Umkristallisation aus Aethanol-Methanol = 1:3 und Trocknen bei 150° am Hochvakuum bei 321-322° (Zers.).

Bei der Umkristallisation des obigen Sulfats aus Wasser erhält man nach Trocknen bei 160° am Hochvakuum ein Sulfat (Kristallmodifikation), das bei 278-280° schmilzt. Dieses kann durch erneute Umkristallisation aus Methanol-Aether wieder in das bei 321-322° schmelzende Sulfat übergeführt werden.

Beispiel 48:

a) Zu einer schwach siedenden Lösung von 17,02 g (0,05 Mol) 6-Phenyl-6-(4-pyridyl)-3,4,6,7-tetrahydro-2H-pyrimido[1,2-c]chinazolin in 150 ml Aethanol gibt man 3,33 ml 15-n. Schwefelsäure (0,025 Mol). Nach Zugabe von Aktivkohle und anschliessender Filtration wird das Filtrat unter Rühren tropfenweise mit 100 ml Aether versetzt. Man lässt langsam auf ca. 5° abkühlen, filtriert das ausgefallene Salz ab und wäscht es mit einem Gemisch Aethanol:Aether = 1:1. Nach

Trocknen über Natriumhydroxid im Hochvakuum bei 160° schmilzt das erhaltene Bis-[6-phenyl-6-(4-pyridyl)-3,4,6,7-tetrahydro-2H-pyrimido-[1,2-c]chinazolin]-sulfat-monohydrat bei 300-303°.

b) Zu einer Suspension von 17,02 g (0,05 Mol) 6-Phenyl-6-(4-pyridyl)-3,4,6,7-tetrahydro-2H-pyrimido[1,2-c]chinazolin in 30 ml Wasser gibt man unter Rühren eine Lösung von 2,55 g 96%iger Schwefelsäure (0,025 Mol) in 10 ml Wasser. Die erhaltene ca. 40° warme Lösung wird filtriert. Das Filtrat lässt man im Verlauf von 4 Stunden langsam auf 0° abkühlen und filtriert dann das ausgefallene Produkt ab. Die Mutterlauge wird am Vakuum eingeengt, wobei man nach Abkühlen und Filtration eine weitere Charge Produkt erhält. Nach Trocknen der vereinigten Chargen bei Raumtemperatur weist das Bis-[6-phenyl-6-(4-pyridyl)-3,4,6,7-tetrahydro-2H-pyrimido[1,2-c]chinazolin]-sulfat einen Wassergehalt von 11,63% auf und schmilzt bei 235-240°. (Proben mit einem Wassergehalt von 8,7% bzw. 7,6% schmelzen ebenfalls bei 235-240°).

Beispiel 49: 1,7 g (0,005 Mol) 6-Phenyl-6-(4-pyridyl)-3,4,6,7-tetrahydro-2H-pyrimido[1,2-c]chinazolin und 0,79 g (0,005 Mol) Benzolsulfonsäure werden in 280 ml Wasser auf 100° erhitzt. Man filtriert dann und lässt das Filtrat auf 5° abkühlen. Das dabei ausgefallene Produkt wird abfiltriert, mit Wasser gewaschen und getrocknet. Das erhaltene Benzolsulfonat-monohydrat zersetzt sich bei 164-166°.

Beispiel 50: Zu einer siedenden Lösung von 8,51 g (0,025 Mol) 6-Phenyl-6-(4-pyridyl)-3,4,6,7-tetrahydro-2H-pyrimido[1,2-c]chinazolin in 70 ml Aethanol gibt man eine Lösung von 3,05 g (0,025 Mol) Benzoesäure in 10 ml Aethanol. Nach Zugabe von 120 ml Aether und Abkühlen auf 5° wird das ausgefallene Produkt abfiltriert, mit einem Gemisch Aethanol:Aether = 2:3 gewaschen und dann getrocknet. Das erhaltene Benzoat schmilzt bei 241-243°.

Beispiel 51: Man erhitzt 3,40 g (0,01 Mol) 6-Phenyl-6-(4-pyridyl)-3,4,6,7-tetrahydro-2H-pyrimido[1,2-c]chinazolin in einer Lösung von 0,72 g (0,012 Mol) Essigsäure in 5 ml Wasser. Nach Abkühlen der Lösung im Eisbad wird das ausgefallene Produkt abfiltriert, mit wenig eiskaltem Wasser gewaschen und getrocknet. Das erhaltene Acetat-dihydrat schmilzt bei ca. 132-137° (mit Sintern ab 120°).

Beispiel 52: Analog Beispiel 12 erhält man unter Verwendung von 3,40 g (0,01 Mol) 6-Phenyl-6-(4-pyridyl)-3,4,6,7-tetrahydro-2H-pyrimido[1,2-c]chinazolin und 1,16 g (0,01 Mol) Fumarsäure das entsprechende Fumarat, welches sich nach Umkristallisation aus Methanol bei etwa 230° zersetzt. Dieses Salz enthält noch 2,9% Wasser.

Bei der Umkristallisation aus Wasser erhält man das 6-Phenyl-6-(4-pyridyl)-3,4,6,7-tetrahydro-2H-pyrimido[1,2-c]chinazolin-1 1/2-fumarat, welches sich bei 232-235° zersetzt.

Beispiel 53: Ein Gemisch von 9,1 g (0,056 Mol) 2-Amino-3-(4,5-dihydro-1H-imidazol-2-yl)-pyridin, 12,5 g (0,084 Mol) Propyl-(4-pyridyl)-keton und 70 g Polyphosphorsäure wird während 18 Stunden bei 150° gerührt. Nach Aufarbeitung analog Beispiel 26 reinigt man das erhaltene Rohprodukt durch Filtration über Kieselgel mit einem Lösungsmittelgemisch Chloroform:Methanol = 9:1. Die das gewünschte Produkt enthaltenden Fraktionen werden eingedampft und der Rückstand aus Aethylacetat umkristallisiert. Das erhaltene 5-Propyl-5-(4-pyridyl)-2,3,5,6-tetrahydro-imidazo[1,2-c]pyrido[3,2-e]pyrimidin schmilzt bei 182-183,5°.

Analog erhält man unter Verwendung von 9,16 g (0,05 Mol) Phenyl-(4-pyridyl)-keton, jedoch bei einer Reaktionsdauer von 24 Stunden bei 140°C und anschliessend 24 Stunden bei 180°C, das 5-Phenyl-5-(4-pyridyl)-2,3,5,6-tetrahydro-imidazo[1,2-c]pyrido[[3,2-e]pyrimidin, welches nach Umkristallisation aus Aethylacetat/Hexan bei 240-241° schmilzt. Das mit Salzsäure in Wasser bereitete Hydrochlorid schmilzt bei 312-314° (Wassergehalt 1,2%).

Das als Ausgangsstoff verwendete 2-Amino-3-(4,5-dihydro-1H-imidazol-2-yl)-pyridin wird wie folgt hergestellt: Ein Gemisch von 23,8 g (0,2 Mol) 2-Amino-3-pyridincarbonitril [J. Heterocyclic Chem. 15, 877-880 (1975)], 24 g (0,4 Mol) Aethylendiamin und 6 Tropfen Schwefelkohlenstoff wird während 2 Stunden bei 150° gerührt und dann im Vakuum eingedampft. Den kristallinen Rückstand nimmt man in 200 ml eiskaltem Aether auf. Nach Filtration der Aetherlösung wird das daraus erhaltene Rohprodukt aus Hexan und Aethanol/Aether umkristallisiert. Das erhaltene 2-Amino-3-(4,5-dihydro-1H-imidazol-2-yl)-pyridin schmilzt bei 155-157°.

Beispiel 54: Ein Gemisch von 14,08 g (0,08 Mol) 4-Amino-3-(1,4,5,6-tetrahydro-2-pyrimidinyl)-pyridin, 17,58 g (0,096 Mol) Phenyl-(4-pyridyl)-keton und 240 g Polyphosphorsäure wird unter Rühren 43 Stunden bei 170°, 17 Stunden bei 180° und 24 Stunden bei 200° erhitzt. Man kühlt ab, versetzt das Reaktionsgemisch mit Eiswasser und einem Ueberschuss an 30%iger Natronlauge, extrahiert mit Chloroform und dampft die organische Phase nach Trocknen über Natriumsulfat im Vakuum ein. Zur Reinigung wird der Rückstand mit einem Lösungsmittelgemisch von Chloroform:Methanol:konz. Ammoniak = 40:10:1 über Kieselgel chromatographiert. Die das gewünschte Produkt enthaltenden Fraktionen werden eingedampft und der Rückstand aus Aethanol/Aethylacetat/Aether umkristallisiert. Das erhaltene 6-Phenyl-6-(4-pyridyl)-3,4,6,7-tetrahydro-2H-pyrido[3,4-e]pyrimido[1,2-c]pyrimidin schmilzt bei 199-201° (Wassergehalt: 3%).

Die Ausgangsverbindung, das 4-Amino-3-(1,4,5,6-tetrahydro-2-pyrimidinyl)-pyridin, wird wie folgt hergestellt:
Ein Gemisch von 35,7 g (0,3 Mol) 4-Amino-3-pyridincarbonitril (US-PS 3 517 021), 44,5 g (95 Mol) Propylendiamin und etwa 10 Tropfen Schwefelkohlenstoff wird während 20 Stunden bei 140° gerührt.
Nach Eindampfen des Reaktionsgemischs im Vakuum kristallisiert man den Rüchstand aus Aethanol um. Das erhaltene 4-Amino-3-(1,4,5,6-tetrahydro-2-pyrimidinyl)-pyridin schmilzt bei 208-210°.

Beispiel 55: Man rührt ein Gemisch von 8,1 g (0,05 Mol) 4-Amino-3-(4,5-dihydro-1H-imidazol-2-yl)-pyridin, 11,2 g (0,075 Mol) Propyl-(4-pyridyl)-keton und 150 g Polyphosphorsäure während 30 Stunden bei 150°, setzt nochmals 5,6 g Propyl-(4-pyridyl)-keton zu und rührt weitere 16 Stunden bei 150°. Nach Aufarbeitung analog Beispiel 26 erhält man das gewünschte 5-Propyl-5-(4-pyridyl)-2,3,5,6-tetrahydro-imidazo-[1,2-c]pyrido[3,4-e]pyrimidin, welches nach Umkristallisation aus Aethylacetat bei 238-240° schmilzt.

Die Ausgangsverbindung, das 4-Amino-3-(4,5-dihydro-1H-imidazol-2-yl)-pyridin, erhält man wie folgt:
Ein Gemisch von 23,8 g (0,2 Mol) 4-Amino-3-pyridincarbonitril, 24 g (0,4 Mol) Aethylendiamin und etwa 8 Tropfen Schwefelkohlenstoff wird während 1,5 Stunden bei 120° gerührt. Man gibt nochmals 15 ml Aethylendiamin zum Reaktionsgemisch, erhitzt es weitere 2 Stunden bei 120° und dampft es dann im Vakuum ein. Nach Umkristallisation aus Aethanol schmilzt das erhaltene 4-Amino-3-(4,5-dihydro-1H-imidazol-2-yl)-pyrimidin bei 198-199°.

Beispiel 56: 8,75 g (0,05 Mol) 2-(2-Aminophenyl)-1,4,5,6-tetrahydro-pyrimiden und 10,4 g (0,05 Mol) Phenyl-styryl-keton (2-Benzylidenacetophenon) werden in eine Lösung von 21,7 g Polyphosphatester in 75 ml Chloroform gegeben und das Reaktionsgemisch 16 Stunden unter Rückfluss gekocht. Der nach Aufarbeitung analog Beispiel 39 erhaltene ölige Rückstand wird mit einem Lösungsmittelgemisch von Chloroform: Methanol:konz. wässrige Ammoniaklösung = 70:30:5 an Kieselgel chromatographiert. Die das gewünschte Produkt enthaltenden Fraktionen werden eingedampft und der Rückstand aus Acetonitril umkristallisiert. Das so erhaltene 6-Phenyl-6-styryl-3,4,6,7-tetrahydro-2H-pyrimido[1,2-c]-chinazolin schmilzt bei 143-145°, nach Trocknen im Hochvakuum bei 100°.

Beispiel 57: Zu einer Suspension von 17,02 g (0,05 Mol) 6-Phenyl-6-(4-pyridyl)-3,4,6,7-tetrahydro-2H-pyrimido[1,2-c]chinazolin in 100 ml Wasser gibt man unter Rühren 50 ml 1-n. Salzsäure und soviel Wasser bis sich beim Erhitzen zum Sieden eine Lösung gebildet hat. Diese wird über Aktivkohle filtriert und das Filtrat bis zur beginnenden Kristallisation eingeengt. Nach Abkühlen auf ca. 5° filtriert man das ausgefallene Salz ab und wäscht es mit wenig eiskaltem Wasser. Das erhaltene 6-Phenyl-6-(4-pyridyl)-3,4,6,7-tetrahydro-2H-pyrimido-[1,2-c]chinazolin-hydrochlorid-dihydrat zersetzt sich bei 345-347°.

Patentansprüche: (für alle benannten Länder ausser Oesterreich)

1. Polyazaheterocyclische Verbindungen der allgemeinen Formel I

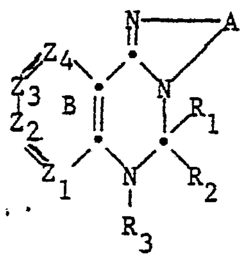

(I),

in welcher $R_1$ und $R_2$ unabhängig voneinander gegebenenfalls substituierte, niedere aliphatische Kohlenwasserstoffreste, gegebenenfalls substituiertes, direkt oder über Niederalkylen oder Niederalkenylen gebundenes Aryl oder Heteroaryl mit höchstens zwei Ringen, $R_3$ Wasserstoff oder Niederalkyl und A gegebenenfalls verzweigtes Niederalkylen oder Niederalkenylen mit 2 bis 4 Kohlenstoffatomen in direkter Kette zwischen den anliegenden Stickstoffatomen bedeuten und $Z_1$, $Z_2$, $Z_3$ und $Z_4$ Glieder des unsubstituierten oder substituierten Ringes B sind und gegebenenfalls entsprechend substituierte Reste -CH= bedeuten, eines jedoch auch den Rest -N= bedeuten kann, wobei $R_1$ und $R_2$ nicht beide Methyl oder beide Aethyl bedeuten, wenn $R_3$ Wasserstoff und A Aethenylen bedeutet und zugleich der Ring B unsubstituiert ist, und ihre Säureadditionssalze.

2. Verbindungen der im Anspruch 1 angegebenen allgemeinen Formel I, in denen $R_1$ und $R_2$ Reste entsprechend der im Anspruch 1 angegebenen Definition bedeuten, die unsubstituiert oder als niedere aliphatische Kohlenwasserstoffreste durch Niederalkoxy, Niederalkylthio oder Niederalkoxycarbonyl und als, bzw. in Aryl- oder Heteroarylresten mit höchstens zwei Ringen durch Niederalkyl, Niederalkoxy, Niederalkylthio, Halogen mit einer Atomnummer von höchstens 35, Trifluormethyl, Methylendioxy, Hydroxy, Sulfamoyl, Nitro oder gegebenenfalls durch Niederalkyl mono- oder disubstituiertes oder durch Niederalkylen oder 3-Oxa-1,5-niederalkylen substituiertes Amino ein- oder mehrfach substituiert sind und zusammen und einschliesslich der gegebenenfalls vorhandenen Substituenten 3 bis 20 Kohlenstoffatome, $R_3$ Wasserstoff oder Niederalkyl mit höchstens 4 Kohlenstoffatomen und A unsubstituiertes oder durch Niederalkyl substituiertes Aethylen oder Trimethylen mit insgesamt höchstens 4 bzw. 5 Kohlenstoffatomen, Aethenylen, Propenylen oder Tetramethylen bedeutet, die Reste $Z_1$, $Z_2$, $Z_3$ und $Z_4$ die unter der Formel I angegebene Bedeutung haben, und der Ring B unsubstituiert ist oder in der vorstehend für Aryl $R_1$ und $R_2$ angegebenen Weise substituiert sein kann, und deren Säureadditionssalze.

3. Verbindungen der im Anspruch 1 angegebenen allgemeinen Formel I, in denen $R_1$ monoheterocyclisches monocyclisches Heteroaryl, das unsubstituiert oder durch Niederalkyl, Niederalkoxy und/oder Halogen bis Atomnummer 35 substituiert ist und über Methylen oder direkt gebunden ist, $R_2$ einen ebensolchen Rest, oder unsubstituiertes oder, wie vorstehend für monocyclisches Heteroaryl $R_1$ angegeben, substituiertes Phenyl oder unsubstituiertes oder, wie im Anspruch 2 angegeben, substituiertes Niederalkyl, $R_3$ Wasserstoff oder Alkyl mit höchstens 4 Kohlenstoffatomen, und A Aethylen oder Trimethylen bedeutet, die Reste $Z_1$, $Z_2$, $Z_3$ und $Z_4$ die im Anspruch 1 angegebene Bedeutung haben und der Ring B unsubstituiert oder in der vorstehend für $R_1$ angegebenen Weise substituiert ist, und deren Säureadditionssalze.

4. Verbindungen der im Anspruch 1 angegebenen allgemeinen Formel I, in denen $R_1$ unsubstituiertes oder durch Niederalkyl, Niederalkoxy und/oder Halogen bis Atomnummer 35 substituiertes Pyridyl, das direkt oder über Methylen gebunden ist, oder unsubstituiertes oder entsprechend substituiertes Thienyl oder Furyl, $R_2$ einen ebensolchen Rest oder unsubstituiertes oder, wie vorstehend für $R_1$ angegeben, substituiertes Phenyl, oder Niederalkyl oder Niederalkoxycarbonylniederalkyl, $R_3$ Wasserstoff oder Alkyl mit höchstens 4 Kohlenstoffatomen, und A Aethylen oder Trimethylen bedeutet, die Reste $Z_1$, $Z_2$, $Z_3$ und $Z_4$ die im Anspruch 1 ange-

BAD ORIGINAL

gebene Bedeutung haben und der Ring B unsubstituiert oder in der vorstehend für $R_1$ angegebenen Weise substituiert ist, und deren Säureadditionssalze.

5. Verbindungen der im Anspruch 1 angegebenen allgemeinen Formel I, in denen $R_1$ unsubstituiertes oder durch Niederalkyl substituiertes Pyridyl, oder Thienyl, $R_2$ einen ebensolchen Rest oder unsubstituiertes oder durch Niederalkyl, Niederalkoxy oder Halogen bis Atomnummer 35 substituiertes Phenyl, oder Niederalkyl oder Niederalkoxycarbonylniederalkyl, $R_3$ Wasserstoff oder Niederalkyl, A Aethylen oder Trimethylen und $Z_1$, $Z_2$, $Z_3$ und $Z_4$ Reste -CH= bedeuten, von denen $Z_2$ durch Halogen bis Atomnummer 35, insbesondere Chlor, substituiert sein kann und somit der Ring B unsubstituiert oder entsprechend substituiert ist, und deren pharmazeutisch annehmbare Säureadditionssalze.

6. Verbindungen der im Anspruch 1 angegebenen allgemeinen Formel I, in denen $R_1$ unsubstituiertes oder durch Methyl substituiertes Pyridyl, oder 2-Thienyl, $R_2$ einen ebensolchen Rest oder unsubstituiertes oder durch Niederalkyl, Niederalkoxy oder Halogen bis Atomnummer 35 substituiertes Phenyl, oder Niederalkyl oder Niederalkoxycarbonylmethyl, $R_3$ Wasserstoff, Methyl oder Aethyl, A Aethylen oder Trimethylen und $Z_1$, $Z_2$, $Z_3$ und $Z_4$ Reste -CH= bedeuten, von denen $Z_2$ durch Chlor, substituiert sein kann und somit der Ring B unsubstituiert oder entsprechend substituiert ist, und deren pharmazeutisch annehmbare Säureadditionssalze.

7. Verbindungen gemäss einem der Ansprüche 3 bis 6, in denen A Aethylen ist, und ihre pharmazeutisch annehmbaren Säureadditionssalze.

8. Verbindungen gemäss einem der Ansprüche 3 bis 6, in denen A Trimethylen ist und ihre pharmazeutisch annehmbaren Säureadditionssalze.

9. 5-Methyl-5-(4-pyridyl)-2,3,5,6-tetrahydroimidazo[1,2-c]chinazolin und seine pharmazeutisch annehmbaren Säureadditionssalze.

10. 5-Methyl-5-(3-methyl-2-pyridyl-2,3,5,6-tetrahydroimidazo[1,2-c]-chinazolin und seine pharmazeutisch annehmbaren Säureadditionssalze.

11. 5-Butyl-5-(4-pyridyl)-2,3,5,6-tetrahydro-imidazo[1,2-c]chinazolin und seine pharmazeutisch annehmbaren Säureadditionssalze.

12. 5-Methyl-5-(2-pyridyl)-8-chlor-2,3,5,6-tetrahydro-imidazo[1,2-c]-chinazolin und seine pharmazeutisch annehmbaren Säureadditionssalze.

13. 5-Phenyl-5-(4-pyridyl)-2,3,5,6-tetrahydro-imidazo[1,2-c]chinazolin und seine pharmazeutisch annehmbaren Säureadditionssalze.

14. 5-(4-Pyridyl)-2,3,5,6-tetrahydro-imidazo[1,2-c]chinazolin-5-essigsäureäthylester und seine pharmazeutisch annehmbaren Säureadditionssalze.

15. 6-Phenyl-6-(4-pyridyl)-3,4,6,7-tetrahydro-2H-pyrimido[1,2-c]-chinazolin und seine pharmazeutisch annehmbaren Säureadditionssalze.

16. 6-Phenyl-6-(2-pyridyl)-3,4,6,7-tetrahydro-2H-pyrimido[1,2-c]-chinazolin und seine pharmazeutisch annehmbaren Säureadditionssalze.

17. 6-(4-Methylphenyl)-6-(4-pyridyl)-3,4,6,7-tetrahydro-2H-pyrimido-[1,2-c]chinazolin und seine pharmazeutisch annehmbaren Säureadditionssalze.

18. 6-(4-Chlorphenyl)-6-(4-pyridyl)-3,4,6,7-tetrahydro-2H-pyrimido-[1,2-c]chinazolin und seine pharmazeutisch annehmbaren Säureadditionssalze.

19. 6-(4-Pyridyl)-6-(2-thienyl)-3,4,6,7-tetrahydro-2H-pyrimido[1,2-c]-chinazolin und seine pharmazeutisch annehmbaren Säureadditionssalze.

20. 7-Aethyl-6-phenyl-6-(4-pyridyl)-3,4,6,7-tetrahydro-2H-pyrimido-[1,2-c]chinazolin und seine pharmazeutisch annehmbaren Säureadditionssalze.

21. Verfahren zur Herstellung der Verbindungen der im Anspruch 1 angegebenen allgemeinen Formel I, in welcher $R_1$, $R_2$, $R_3$, $Z_1$, $Z_2$, $Z_3$ und $Z_4$ und A die dort angegebene Bedeutung haben, und der Ring B, wie dort angegeben, substituiert sein kann, und ihrer Säureadditionssalze, dadurch gekennzeichnet, dass man eine Verbindung der allgemeinen Formel II

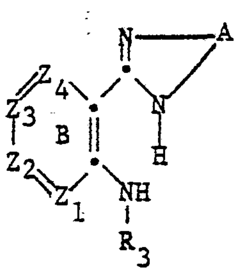

(II)

in welcher $R_3$, $Z_1$, $Z_2$, $Z_3$, $Z_4$ und A die im Anspruch 1 angegebene Bedeutung haben und der Ring B, wie dort angegeben, substituiert sein kann, oder ein Säureadditonssalz derselben mit einem Keton der allgemeinen Formel III,

$$R_1 - CO - R_2 \qquad \text{(III)}$$

in welcher $R_1$ und $R_2$ die im Anspruch 1 angegebene Bedeutung haben, oder einem reaktionsfähigen funktionellen Derivat desselben kondensiert, gewünschtenfalls in eine Verbindung der allgemeinen Formel I, in welcher $R_3$ Wasserstoff bedeutet, Niederalkyl als Rest $R_3$ einführt, und/oder eine erhaltene Verbindung der allgemeinen Formel I in ein Säureadditionssalz überführt oder aus einem erhaltenen Säureadditionssalz die Verbindung der allgemeinen Formel I freisetzt.

22. Die nach dem Verfahren gemäss Anspruch 21 erhältlichen Verbindungen und ihre Säureadditionssalze.

23. Pharmazeutische Präparate, gekennzeichnet durch einen Gehalt an einer Verbindung der allgemeinen Formel I gemäss einem der Ansprüche 1 bis 6 oder einem pharmazeutisch annehmbaren Säureadditionssalz derselben, und mindestens einem pharmazeutischen Trägerstoff.

24. Pharmazeutische Präparate, gekennzeichnet durch einen Gehalt an einer Verbindung der allgemeinen Formel I gemäss einem der Ansprüche 9 bis 14 oder einem pharmazeutisch annehmbaren Säureadditonssalz derselben, und mindestens einem pharmazeutischen Trägerstoff.

25. Pharmazeutische Präparate, gekennzeichnet durch einen Gehalt an einer Verbindung der allgemeinen Formel I gemäss einem der Ansprüche 15 bis 20 oder einem pharmazeutisch annehmbaren Säureadditionssalz derselben, und mindestens einem pharmazeutischen Trägerstoff.

26. Verbindungen der allgemeinen Formel I gemäss einem der Ansprüche 1 bis 20 und pharmazeutisch annehmbare Säureadditionssalze derselben zur Anwendung in einem Verfahren zur therapeutischen Behandlung des menschlichen oder tierischen Körpers.

27. Verbindungen der allgemeinen Formel I gemäss Anspruch 1, in denen A gegebenenfalls verzweigtes Niederalkylen oder Niederalkenylen mit 2 Kohlenstoffatomen in direkter Kette zwischen den anliegenden Stickstoffatomen bedeutet, oder gemäss einem der Ansprüche 7 und 9 bis 14, und pharmazeutisch annehmbare Säureadditionssalze derselben zur Anwendung als Antidiabetica.

28. Verbindungen der allgemeinen Formel I gemäss Anspruch 1, in denen A gegebenenfalls verzweigtes Niederalkylen oder Niederalkenylen mit 3 oder 4 Kohlenstoffatomen in direkter Kette zwischen den anliegenden

Stickstoffatomen bedeutet, oder gemäss einem der Ansprüche 8 und 15 bis 20, und pharmazeutisch annehmbare Säureadditionssalze derselben zur Anwendung als Diuretica und Antihypertensiva.

Patentansprüche: (für Oesterreich)

1. Verfahren zur Herstellung von neuen polyazaheterocyclischen Verbindungen der allgemeinen Formel I

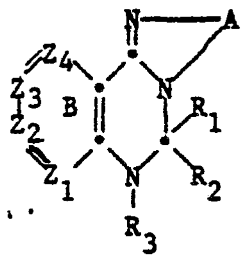

(I),

in welcher $R_1$ und $R_2$ unabhängig voneinander gegebenenfalls substituierte, niedere aliphatische Kohlenwasserstoffreste, gegebenenfalls substituiertes, direkt oder über Niederalkylen oder Niederalkenylen gebundenes Aryl oder Heteroaryl mit höchstens zwei Ringen, $R_3$ Wasserstoff oder Niederalkyl und A gegebenenfalls verzweigtes Niederalkylen oder Niederalkenylen mit 2 bis 4 Kohlenstoffatomen in direkter Kette zwischen den anliegenden Stickstoffatomen bedeuten und $Z_1$, $Z_2$, $Z_3$ und $Z_4$ Glieder des unsubstituierten oder substituierten Ringes B sind und gegebenenfalls entsprechend substituierte Reste -CH= bedeuten, eines jedoch auch den Rest -N= bedeuten kann, wobei $R_1$ und $R_2$ nicht beide Methyl oder beide Aethyl bedeuten, wenn $R_3$ Wasserstoff und A Aethenylen bedeutet und zugleich der Ring B unsubstituiert ist, und ihrer Säureadditionssalze, dadurch gekennzeichnet, dass man eine Verbindung der allgemeinen Formel II

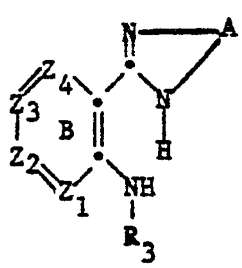

(II)

BAD ORIGINAL

in welcher $R_3$, $Z_1$, $Z_2$, $Z_3$, $Z_4$ und A die unter der Formel I angegebene Bedeutung haben und der Ring B, wie dort angegeben, substituiert sein kann, oder ein Säureadditonssalz derselben mit einem Keton der allgemeinen Formel III,

$$R_1 - CO - R_2 \quad \text{(III)}$$

in welcher $R_1$ und $R_2$ die unter der Formel I angegebene Bedeutung haben, oder einem reaktionsfähigen funktionellen Derivat desselben kondensiert, gewünschtenfalls in eine Verbindung der allgemeinen Formel I, in welcher $R_3$ Wasserstoff bedeutet, Niederalkyl als Rest $R_3$ einführt, und/oder eine erhaltene Verbindung der allgemeinen Formel I in ein Säureadditionssalz überführt oder aus einem erhaltenen Säureadditionssalz die Verbindung der allgemeinen Formel I freisetzt.

2. Verfahren nach Anspruch 1, dadurch gekennzeichnet, dass man Verbindungen der im Anspruch 1 angegebenen allgemeinen Formel I, in denen $R_1$ und $R_2$ Reste entsprechend der im Anspruch 1 angegebenen Definition bedeuten, die unsubstituiert oder als niedere aliphatische Kohlenwasserstoffreste durch Niederalkoxy, Niederalkylthio oder Niederalkoxycarbonyl und als, bzw. in Aryl- oder Heteroarylresten mit höchstens zwei Ringen durch Niederalkyl, Niederalkoxy, Niederalkylthio, Halogen mit einer Atomnummer von höchstens 35, Trifluormethyl, Methylendioxy, Hydroxy, Sulfamoyl, Nitro oder gegebenenfalls durch Niederalkyl mono- oder disubstituiertes oder durch Niederalkylen oder 3-Oxa-1,5-niederalkylen substituiertes Amino ein- oder mehrfach substituiert sind und zusammen und einschliesslich der gegebenenfalls vorhandenen Substituenten 3 bis 20 Kohlenstoffatome, $R_3$ Wasserstoff oder Niederalkyl mit höchstens 4 Kohlenstoffatomen und A unsubstituiertes oder durch Niederalkyl substituiertes Aethylen oder Trimethylen mit insgesamt höchstens 4 bzw. 5 Kohlenstoffatomen, Aethenylen, Propenylen oder Tetramethylen bedeutet, die Reste $Z_1$, $Z_2$, $Z_3$ und $Z_4$ die unter der For-

mel I angegebene Bedeutung haben, und der Ring B unsubstituiert ist oder in der vorstehend für Aryl $R_1$ und $R_2$ angegebenen Weise substituiert sein kann, oder deren Säureadditionssalze herstellt.

3. Verfahren nach Anspruch 1, dadurch gekennzeichnet, dass Verbindungen der im Anspruch 1 angegebenen allgemeinen Formel I, in denen $R_1$ monoheterocyclisches monocyclisches Heteroaryl, das unsubstituiert oder durch Niederalkyl, Niederalkoxy und/oder Halogen bis Atomnummer 35 substituiert ist und über Methylen oder direkt gebunden ist, $R_2$ einen ebensolchen Rest, oder unsubstituiertes oder, wie vorstehend für monocyclisches Heteroaryl $R_1$ angegeben, substituiertes Phenyl oder unsubstituiertes oder, wie im Anspruch 2 angegeben, substituiertes Niederalkyl, $R_3$ Wasserstoff oder Alkyl mit höchstens 4 Kohlenstoffatomen, und A Aethylen oder Trimethylen bedeutet, die Reste $Z_1$, $Z_2$, $Z_3$ und $Z_4$ die im Anspruch 1 angegebene Bedeutung haben und der Ring B unsubstituiert oder in der vorstehend für $R_1$ angegebenen Weise substituiert ist, oder deren Säureadditionssalze herstellt.

4. Verfahren nach Anspruch 1, dadurch gekennzeichnet, dass man Verbindungen der im Anspruch 1 angegebenen allgemeinen Formel I, in denen $R_1$ unsubstituiertes oder durch Niederalkyl, Niederalkoxy und/oder Halogen bis Atomnummer 35 substituiertes Pyridyl, das direkt oder über Methylen gebunden ist, oder unsubstituiertes oder entsprechend substituiertes Thienyl oder Furyl, $R_2$ einen ebensolchen Rest oder unsubstituiertes oder, wie vorstehend für $R_1$ angegeben, substituiertes Phenyl, oder Niederalkyl oder Niederalkoxycarbonylniederalkyl, $R_3$ Wasserstoff oder Alkyl mit höchstens 4 Kohlenstoffatomen, und A Aethylen oder Trimethylen bedeutet, die Reste $Z_1$, $Z_2$, $Z_3$ und $Z_4$ die im Anspruch 1 angegebene Bedeutung haben und der Ring B unsubstituiert oder in der vorstehend für $R_1$ angegebenen Weise substituiert ist, oder deren Säureadditionssalze herstellt.

5. Verfahren nach Anspruch 1, dadurch gekennzeichnet, dass man Verbindungen der im Anspruch 1 angegebenen allgemeinen Formel I, in denen $R_1$ unsubstituiertes oder durch Niederalkyl substituiertes Pyridyl, oder Thienyl, $R_2$ einen ebensolchen Rest oder unsubstituiertes oder durch Niederalkyl, Niederalkoxy oder Halogen bis Atomnummer 35 substituiertes Phenyl, oder Niederalkyl oder Niederalkoxycarbonylniederalkyl, $R_3$ Wasserstoff oder Niederalkyl, A Aethylen oder Trimethylen und $Z_1$, $Z_2$, $Z_3$ und $Z_4$ Reste -CH= bedeuten, von denen $Z_2$ durch Halogen bis Atomnummer 35, insbesondere Chlor, substituiert sein kann und somit der Ring B unsubstituiert oder entsprechend substituiert ist, oder deren pharmazeutisch annehmbare Säureadditionssalze herstellt.

6. Verfahren nach Anspruch 1, dadurch gekennzeichnet, dass man Verbindungen der im Anspruch 1 angegebenen allgemeinen Formel I, in denen $R_1$ unsubstituiertes oder durch Methyl substituiertes Pyridyl, oder 2-Thienyl, $R_2$ einen ebensolchen Rest oder unsubstituiertes oder durch Niederalkyl, Niederalkoxy oder Halogen bis Atomnummer 35 substituiertes Phenyl, oder Niederalkyl oder Niederalkoxycarbonylmethyl, $R_3$ Wasserstoff, Methyl oder Aethyl, A Aethylen oder Trimethylen und $Z_1$, $Z_2$, $Z_3$ und $Z_4$ Reste -CH= bedeuten, von denen $Z_2$ durch Chlor, substituiert sein kann und somit der Ring B unsubstituiert oder entsprechend substituiert ist, oder deren pharmazeutisch annehmbare Säureadditionssalze herstellt.

7. Verfahren gemäss einem der Ansprüche 3 bis 6, dadurch gekennzeichnet, dass man entsprechende Verbindungen der allgemeinen Formel I, in denen A Aethylen ist, oder ihre pharmazeutisch annehmbaren Säureadditionssalze herstellt.

8. Verfahren gemäss einem der Ansprüche 3 bis 6, dadurch gekennzeichnet, dass man entsprechende Verbindungen der allgemeinen Formel I, in denen A Trimethylen ist, oder ihre pharmazeutisch annehmbaren Säureadditionssalze herstellt.

9. Verfahren gemäss Anspruch 1, dadurch gekennzeichnet, dass man das 5-Methyl-5-(4-pyridyl)-2,3,5,6-tetrahydro-imidazo[1,2-c]chinazolin oder seine pharmazeutisch annehmbaren Säureadditionssalze herstellt.

10. Verfahren gemäss Anspruch 1, dadurch gekennzeichnet, dass man das 5-Methyl-5-(3-methyl-2-pyridyl-2,3,5,6-tetrahydro-imidazo[1,2-c]-chinazolin oder seine pharmazeutisch annehmbaren Säureadditionssalze herstellt.

11. Verfahren gemäss Anspruch 1, dadurch gekennzeichnet, dass man das 5-Butyl-5-(4-pyridyl)-2,3,5,6-tetrahydro-imidazo[1,2-c]chinazolin oder seine pharmazeutisch annehmbaren Säureadditionssalze herstellt.

12. Verfahren gemäss Anspruch 1, dadurch gekennzeichnet, dass man das 5-Methyl-5-(2-pyridyl)-8-chlor-2,3,5,6-tetrahydro-imidazo[1,2-c]-chinazolin oder seine pharmazeutisch annehmbaren Säureadditionssalze herstellt.

13. Verfahren gemäss Anspruch 1, dadurch gekennzeichnet, dass man das 5-Phenyl-5-(4-pyridyl)-2,3,5,6-tetrahydro-imidazo[1,2-c]chinazolin oder seine pharmazeutisch annehmbaren Säureadditionssalze herstellt.

14. Verfahren gemäss Anspruch 1, dadurch gekennzeichnet, dass man den 5-(4-Pyridyl)-2,3,5,6-tetrahydro-imidazo[1,2-c]chinazolin-5-essigsäureäthylester oder seine pharmazeutisch annehmbaren Säureadditionssalze herstellt.

15. Verfahren gemäss Anspruch 1, dadurch gekennzeichnet, dass man das 6-Phenyl-6-(4-pyridyl)-3,4,6,7-tetrahydro-2H-pyrimido[1,2-c]chinazolin oder seine pharmazeutisch annehmbaren Säureadditionssalze herstellt.

16. Verfahren gemäss Anspruch 1, dadurch gekennzeichnet, dass man das 6-Phenyl-6-(2-pyridyl)-3,4,6,7-tetrahydro-2H-pyrimido[1,2-c]chinazolin oder seine pharmazeutisch annehmbaren Säureadditionssalze herstellt.

17. Verfahren gemäss Anspruch 1, dadurch gekennzeichnet, dass man das 6-(4-Methylphenyl)-6-(4-pyridyl)-3,4,6,7-tetrahydro-2H-pyrimido[1,2-c]-chinazolin oder seine pharmazeutisch annehmbaren Säureadditionssalze herstellt.

18. Verfahren gemäss Anspruch 1, dadurch gekennzeichnet, dass man das 6-(4-Chlorphenyl)-6-(4-pyridyl)-3,4,6,7-tetrahydro-2H-pyrimido[1,2-c]-chinazolin oder seine pharmazeutisch annehmbaren Säureadditionssalze herstellt.

19. Verfahren gemäss Anspruch 1, dadurch gekennzeichnet, dass man das 6-(4-Pyridyl)-6-(2-thienyl)-3,4,6,7-tetrahydro-2H-pyrimido[1,2-c]-chinazolin oder seine pharmazeutisch annehmbaren Säureadditionssalze herstellt.

20. Verfahren gemäss Anspruch 1, dadurch gekennzeichnet, dass man das 7-Aethyl-6-phenyl-6-(4-pyridyl)-3,4,6,7-tetrahydro-2H-pyrimido[1,2-c]-chinazolin oder seine pharmazeutisch annehmbaren Säureadditionssalze herstellt.

0046446

Nummer der Anmeldung

Europäisches Patentamt

# EUROPÄISCHER RECHERCHENBERICHT

EP 81 81 0320.2

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | Chemical Abstracts Band 83, Nr. 23, 8. Dezember 1975<br>Columbus, Ohio, USA<br>M. CARDELLINI et al. "Derivatives of imidazo [1,2-c] quinazoline and their inhibitory action on platelet aggregation"<br>Seite 14, Spalte 1, Abstract Nr. 188196j<br>& Farmaco, Ed. Sci, Band 30, Nr. 7, 1975, Seiten 536 bis 546<br>-- | 1 | C 07 D 487/04<br>C 07 D 471/14<br>A 61 K 31/415<br>A 61 K 31/505<br>//(C 07 D 487/04, 239/00, 235/00)<br>(C 07 D 487/04, 239/00, 239/00)<br>(C 07 D 471/14, 239/00,235/00, 221/00) |
| | | | RECHERCHIERTE SACHGEBIETE (Int. Cl.³) |
| | DE - A - 1 470 432 (A. WANDER AG)<br>* Anspruch 2 *<br>-- | 21 | A 61 K 31/00<br>C 07 D 471/14<br>C 07 D 487/04 |
| A | US - A - 3 329 679 (T.S. SULKOWSKI et al.)<br>---- | | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 27-10-1981 | FROELICH |